# EUROPEAN PATENT APPLICATION

(11) **EP 3 904 518 A1**
(43) Date of publication of application: **03.11.2021**
(21) Application number: 20763447.8
(22) Date of filing: 25.02.2020
(51) Int. Cl.: C12N 15/113, C12N 9/12

(54) **PHARMACEUTICAL COMPOSITION FOR PREVENTING OR TREATING CANCER, COMPRISING TUT4/7 EXPRESSION MODULATOR**

(30) Priority: 26.02.2019 KR 20190022551
(71) Applicant: Seoul National University R & DB Foundation, Seoul 08826 (KR); Institute for Basic Science, Yuseong-gu Daejeon 34126 (KR)
(72) Inventor: KIM, V. Narry, Seoul 08826 (KR); KIM, Haedong, Seoul 08826 (KR); KIM, Jimi, Seoul 08826 (KR)
(74) Representative: J A Kemp LLP
(86) International application number: PCT/KR2020/002709
(87) International publication number: WO 2020/175898

(57) **Abstract**

The present invention relates to a pharmaceutical composition for preventing or treating cancer, comprising a TUT4/7 expression modulator, wherein the pharmaceutical composition of the present invention can prevent cell division and hinder cancer development by inhibiting the function of TUT4/7, and can increase the amount of miR-324-5p and suppress the function of miR-324-3p.1, and therefore can be effectively used for prevention, treatment, or diagnosis of cancer.

## Description

### Technical Field

The present invention relates to a pharmaceutical composition comprising a TUT4/7 expression regulator for prevention or treatment of cancer and, more specifically, to a pharmaceutical composition for preventing or treating cancer, comprising a nucleic acid regulatory of TUT4/7 expression.

The present invention was made with the support of the Ministry of Health and Welfare, Republic of Korea, under Project No. 1711079098, which was conducted by the Institute of Basic Science in the research program named "Study on the cellular fate decision by RNA" as a branch of the research project titled "Operation Management and Support for Institute of Basic Science", under the research management of the Institute of Basic Science, from 01 January 2018 to 31 December 2018.

This present application claims the benefit of Korean Patent Application No. 10-2019-0022551, filed to the Korean Intellectual Property Office on 26 February 2019, the content of which is herein incorporated by reference in its entirety.

### Background Art

The biogenesis of microRNA (miRNA) is achieved through the cleavage actions of Drosha and Dicer to generate a miRNA duplex, followed by selecting one of the two constituent strands (5p and 3p). The strand selection process of determining which strand is selected is of biological significance because the two strands are definitely different in sequence and can thus regulate the expression of respective different genes.

Meanwhile, the dominantly selected strand may change depending on cellular contexts, which is called alternative strand selection (or "arm switching"). Alternative strand selection can affect tissue differentiation and cancer development. However, the molecular mechanism, such as regulatory mechanism, etc., and physiological significance of such alternative strand selection remain elusive.

### Detailed Description of the Invention

### Technical Problem

The present inventors find that an uridylation by TUT4 and TUT7 (TUT4/7) shifts the cleavage site of Dicer on the pre-mir-324 to produce three functionally different microRNAs (one 5p and two 3p isoforms) and prevents cell division and inhibits cancer development by downregulating of TUT4/7, which is in the same effect as that obtained when the level of miR-324-5p is increased and miR-324-3p.1 is downregulated, and thus completed the present disclosure.

Therefore, an objective of the present invention is to provide a pharmaceutical composition for preventing or treating cancer, comprising: a microRNA (miRNA) comprising the nucleotide sequences of SEQ ID NOS: 1 and 2; a nucleic acid composed of the nucleotide sequence of SEQ ID NO: 4; and a small interfering RNA (siRNA) comprising at least one nucleotide sequence selected from the group consisting of SEQ ID NOS: 5 to 20.

Another objective of the present invention is to provide a method for treating cancer, using: the composition comprising a microRNA (miRNA) comprising the nucleotide sequences of SEQ ID NOS: 1 and 2; a nucleic acid composed of the nucleotide sequence of SEQ ID NO: 4; and a small interfering RNA (siRNA) comprising at least one nucleotide sequence selected from the group consisting of SEQ ID NOS: 5 to 20.

A further objective of the present invention is to provide the pharmaceutical composition for use in treating cancer, the composition comprising a microRNA (miRNA) comprising the nucleotide sequences of SEQ ID NOS: 1 and 2; a nucleic acid composed of the nucleotide sequence of SEQ ID NO: 4; and a small interfering RNA (siRNA) comprising at least one nucleotide sequence selected from the group consisting of SEQ ID NOS: 5 to 20.

A still further objective of the present invention is to provide a composition for diagnosing cancer, comprising an agent for measuring a miRNA expression level of miR-324-5p or miR-324-3p.1.

A still another objective of the present invention is to provide a kit for diagnosing cancer, comprising an agent for measuring a miRNA expression level of miR-324-5p or miR-324-3p.1.

A yet another objective of the present invention is to provide a composition for diagnosing cancer, comprising an agent for measuring a mRNA or protein level of terminal uridylyl transferases 4 (TUT4) or terminal uridylyl transferases 7 (TUT7).

A yet further objective of the present invention is to provide a kit for diagnosing cancer, comprising an agent for measuring a mRNA or protein level of TUT4 or TUT7.

### Technical Solution

The present inventors find that an uridylation by TUT4 and TUT7 (TUT4/7) shifts the cleavage site of Dicer on the pre-mir-324 to produce three functionally different microRNAs (one 5p and two 3p isoforms) and prevents cell division and inhibits cancer development by downregulating of TUT4/7, which is in the same effect as that obtained when the level of miR-324-5p is increased and miR-324-3p.1 is downregulated.

Below, a detailed description will be given of the present invention.

An aspect of the present invention relates to a pharmaceutical composition for preventing or treating cancer, comprising at least one selected from the group consisting of:
a microRNA (miRNA) comprising the nucleotide sequences of SEQ ID NOS: 1 and 2;
a nucleic acid composed of the nucleotide sequence of SEQ ID NO: 4; and
a small interfering RNA (siRNA) comprising at least one nucleotide sequence selected from the group consisting of SEQ ID NOS: 5 to 20.

The cancer may be encephaloma, colon cancer, large intestine cancer, lung cancer, liver cancer, stomach cancer, esophageal cancer, pancreatic cancer, gallbladder cancer, kidney cancer, bladder cancer, prostate cancer, testis cancer, uterine cervical cancer, endometrial cancer, choriocarcinoma, ovarian cancer, breast cancer, thyroid cancer, brain cancer, head and neck cancer, and/or malignant melanoma, but is not limited thereto.

As used herein, the term "microRNA (miRNA)" refers to a non-coding RNA molecule about 22 nucleotides long that functions in post-transcriptional regulation of gene expression by forming base pairs with the 3' untranslated region (UTR) of mRNAs.

The microRNA of the present invention may include the nucleotide sequences of SEQ ID NOS: 1 and 2.

In detail, the microRNA (5p duplex miRNA) may be a duplex in which a microRNA (miR-324-5p) comprising the nucleotide sequence of SEQ ID NO:1 and a microRNA (miR-324-3p.2) comprising the nucleotide sequence of SEQ ID NO: 2 are bound to each other.

The nucleotide sequences of SEQ ID NOS: 1 and 2 may each have a phosphate at the 5' end thereof (5phos).

As used herein, "nucleic acid" is meant to include any DNA or RNA, for example, chromosomal, mitochondrial, viral, and/or bacterial nucleic acid present in tissue sample. It encompasses either or both strands of a double stranded nucleic acid molecule and includes any fragment or portion of an intact nucleic acid molecule.

The nucleic acid of the present invention is construed to include chemically modified DNA or RNA. A person skilled in the art might synthesize or modify nucleic acids in a desired manner using a method known in the art.

The nucleic acid of the present invention may be composed of the nucleotide sequence of SEQ ID NO: 4 (CAGCAGCACCTGGGGCAG) .

As used herein, the term "small interfering RNA (siRNA)" refers to a double-stranded RNA fragment 18-23 nucleotides in length, produced from double-stranded RNAs by catalysis of Dicer, functioning to bind to a mRNA having a complementary sequence to interfere with the expression of the corresponding protein.

The siRNA of the present invention is construed to encompass a chemically modified siRNA which is prevented from being rapid degraded by nucleases in vivo. A person skilled in the art might synthesize and modify siRNA in a desired manner using a method known in the art.

The composition of the present invention may be introduced into cells, together a nucleic acid carrier for increasing intracellular delivery yield.

The small interfering RNA of the present invention may include at least one of the nucleotide sequences of SEQ ID NOS: 5 to 20.

In detail, the small interfering RNA (siTUT4) may be a complex in which a small interfering RNA comprising any one of the nucleotide sequences of SEQ ID NOS: 5 to 8 and a small interfering RNA comprising any one of the nucleotide sequences of SEQ ID NOS: 9 to 12 bind to each other.

Moreover, in detail, the small interfering RNA (siTUT7) may be a complex in which a small interfering RNA comprising any one of the nucleotide sequences of SEQ ID NOS: 13 to 16 and a small interfering RNA comprising any one of the nucleotide sequences of SEQ ID NOS: 17 to 20 bind to each other.

As used in the context of using the miRNA, nucleic acid, and/or siRNA in the present invention, the term "therapeutically effective amount" means an amount required for bringing about a desired result in cancer therapy. Hence, the amount may vary depending on various factors including the kind and severity of the disease to be treated, the kind of the nucleic acid to be administered, the kind of the formulation to be used, the patient's age, body weight, general health state, sex, and diet, the time of administration, the route of administration, the duration of the treatment, and other drugs, such as chemicals used in combination with the composition.

The composition of the present invention may be administered via various routes, such as orally, transdermally, subcutaneously, intravenously, or intramuscularly, and so on. To this end, other additives may be additionally included in the miRNA, nucleic acid and/or siRNA according to the present invention.

The composition of the present invention may be in the form of a tablet, a pill, a powder, a sachet, an elixir, a suspension, an emulsion, a liquid, a syrup, an aerosol, a capsule, a sterile injection, a sterile powder, and so on, and may be preferably formulated into a form suitable for intravenous injection, subcutaneous injection, intradermal injection, intramuscular injection, and the like.

The pharmaceutical composition of the present invention may be formulated as general formulations using the pharmaceutically acceptable carriers and/or excipients according to methods easily practiced by a person skilled in the art to which the present invention pertains, to be prepared as a unit dosage form or to be prepared by introducing the composition into a multi-dosage container. The general formulation refers to a solution in an oil or aqueous medium, a suspension, an emulsion, an extract, a powder, a suppository, a granule, a tablet, or capsule, and may further contain a dispersant or a stabilizer.

A pharmaceutically acceptable carrier, which may be contained in the pharmaceutical composition of the present invention, is one typically used in drug preparation and includes, but not limited to, lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may further include a lubricant, a humectant, a sweetening agent, a favoring agent, an emulsifier, a suspending agent, and a preserving agent besides the components above. A suitable pharmaceutically acceptable carrier and a formulation are described in *Remington's Pharmaceutical Sciences* (19^{th} ed., 1995) .

The pharmaceutical composition of the present invention may be administered orally or parenterally, for example, intravenously, subcutaneously, intramuscularly, intraperitoneally, topically, intranasally, intrapulmonarily, intrarectally, intrathecally, intraocularly, dermally, and transdermally.

A suitable dose of the pharmaceutical composition of the present invention may vary depending on pharmaceutical formulation methods, administration methods, the patient's age, body weight, sex, severity of diseases, diet, administration time, administration route, an excretion rate, and sensitivity for a used pharmaceutical composition. Physicians with average skill may easily determine and diagnose dosage levels of medicine effective for treating or preventing target disorders or diseases.

Another aspect of the present invention pertains to a composition for diagnosing cancer, comprising an agent for measuring an expression level of at least one miRNA selected from the group consisting of miR-324-5p and miR-324-3p.1.

The microRNA (miR-324-5p) may include the nucleotide sequence of SEQ ID NO: 1 and the microRNA (miR-324-3p.1) may include the nucleotide sequence of SEQ ID NO: 3 (ACUGCCCCAGGUGCUGCUGG).

In detail, the microRNA (miR-324-3p.1) includes the nucleotide sequence of SEQ ID NO: 3, with one or more uracil (U) residues further added to the 3' end thereof.

Moreover, the microRNAs may be produced by cleaving from a transcript made in the miR324 gene (GenBank Number: 442898) by Drosha and Dicer.

As used herein, the term "diagnosis" is meant to include determining the susceptibility of a subject to a specific disease or disorder, determining whether a subject currently has a specific disease or disorder, determining the prognosis of a subject suffering from a specific disease or disorder, or performing therametrics (for example, monitoring the status of a subject in order to provide information about therapeutic efficacy).

As used herein, the term "subject" or "patient" means any single entity requiring treatment, including a human, a cow, a dog, a guinea pig, a rabbit, a chicken, an insect and the like, and is intended to encompass any subject that participates in a clinical study test, with no disease clinical findings found therein or which participates in epidemiological studies or used as a control group.

Unless otherwise specified, the term "measuring an expression level of miRNA", as used herein, refers to detecting a target of interest in a corresponding sample.

In the present invention, the target of interest is a corresponding miRNA in a sample. In other words, the detection of the miRNA allows the onset of the cancer to be determined.

For example, an expression level of miRNA may be measured in a biological sample from a patient suspicious of cancer.

In the present invention, miR-324-5p and miR-324-3p.1 are used as diagnostic markers for determining the onset of cancer on the basis of the finding that a subject with cancer exhibits a significant decrease in the expression level of miR-324-5p and a significant increase in the expression level of miR-324-3p.1 in vivo, compared to a subject without cancer.

In the present invention, the agent for measuring an expression level of miRNA refers to a molecule which is useful for detecting the marker miRNA by determining an expression level of the miRNA whose level is decreased or increased in biological specimens from a subject affected with cancer.

In detail, the agent for measuring an expression level of miRNA may be a primer or probe binding specifically to the miRNA.

That is, the detection of nucleic acid may be performed by an amplification reaction using one or more oligonucleotide primers which are hybridized to a nucleic acid molecule encoding the gene or a complementary material to the nucleic acid molecule.

For example, the detecting of the mRNA with a primer may be performed by amplifying a gene sequence using an amplification method such as PCR, followed by verifying the amplification of the gene in a known method in the art.

The term "primer" means a short nucleic acid sequence having a free 3'-OH group, which forms base pairs with a complementary template and serves as a starting point for template strand replication. The primer is able to initiate DNA synthesis in the presence of four different nucleoside triphosphates and an agent for polymerization (i.e., DNA polymerase or reverse transcriptase) in an appropriate buffer and at a suitable temperature. In the present invention, PCR amplification is performed using a sense and an antisense primer which bind specifically to at least one of the miRNAs and the expression level is identified to diagnose the onset of cancer. Modifications may be given PCR conditions and lengths of sense and antisense primers with reference to knowledge known in the art.

The term "probe" refers to a nucleic acid fragment, such as fragments of RNA or DNA, with a label given thereto. A probe may be fabricated into the form of an oligonucleotide, a single stranded DNA, a double stranded DNA, an RNA, and so on. In the present invention, the onset of cancer can be diagnosed by hybridizing one or more of the miRNAs with a probe complementary thereto and determining the expression level of the miRNAs. With reference to knowledge in the art, suitable probes and hybridization conditions can be selected and modified.

Such primers or probes can be appropriately designed by a person skilled in the art on the basis of known sequences.

For example, primers or probes may be chemically synthesized using a phosphoramidite solid support method or other well-known methods. Such nucleic acid sequences may also be modified by various means known in the art. Non-limiting examples of such modifications include methylation, "capping", substitution of one or more analogues of natural nucleotides, and nucleotide variation, for example, variation to non-charged linkages (for example: methyl phosphonate, phosphotriester, phosphoroamidate, carbamates, etc.) or charged linkages (for example: phosphorothioate, phosphorodithioate, etc.).

An expression level of miRNA may be measured using a typical method in the bio-kit field. Examples of the method include reverse transcription polymerase chain reaction (RT-PCR), competitive RT-PCR, real-time RT-PCR, RNase protection assay (RPA), northern blotting, and a gene chip, but are not limited thereto.

Still another aspect of the present invention pertains to a kit for diagnosing cancer, comprising the composition for diagnosing cancer.

The kit may comprise a generally used tool and reagent suitable for use in a kit for diagnosing cancer in addition to the agent for measuring an expression level of miRNA.

Examples of the tool/reagent may include, but are not be limited to, a suitable carrier, a labeling substance capable of generating a detectable signal, a chromophore, a solubilizer, a detergent, a buffer, a stabilizer, and the like. When the labeling substance is an enzyme, the tool and reagent may comprise a substrate capable of measuring enzyme activity and a reaction stopping agent. The carrier may be soluble or insoluble, as exemplified by the known physiologically acceptable buffer PBS for the soluble carrier and by polystyrene, polyethylene, polypropylene, polyester, polyacrylonitrile, a fluorine resin, crosslinked dextran, a polysaccharide, a polymer such as a magnetic microbead made of latex coated with a metal, paper, glass, metals, agarose, and combinations thereof.

For instance, the kit for diagnosing of the present invention may be a kit including an element essential for performing RT-PCR. The RT-PCR kit may contain a test tube or another suitable container, a reaction buffer (various pH values and magnesium concentrations), deoxynucleotides (dNTPs), enzymes such as Taq-polymerase and reverse transcriptase, DNase and RNase inhibitors, DEPC-water, sterile water, and the like.

Since the kit of the present invention comprises the composition as a constituent thereof, descriptions of overlapping contents therebetween will be omitted to avoid excessive complexities.

Still another aspect of the present invention pertains to a composition for diagnosing cancer, comprising an agent for measuring an mRNA or protein level of at least one selected from the group consisting of TUT4 (terminal uridylyl transferases 4) (GenBank Number: 23318) and TUT7 (terminal uridylyl transferases 7) (GenBank Number: 79670).

The TUT4 gene has the nucleotide sequence of SEQ ID NO: 21 and the TUT7 gene has the nucleotide sequence of SEQ ID NO: 22.

As used herein, the term "diagnosis" is meant to include determining the susceptibility of a subject to a specific disease or disorder, determining whether a subject currently has a specific disease or disorder, determining the prognosis of a subject suffering from a specific disease or disorder, or performing therametrics (for example, monitoring the status of a subject in order to provide information about therapeutic efficacy).

As used herein, the term "subject" or "patient" means any single entity requiring treatment, including a human, a cow, a dog, a guinea pig, a rabbit, a chicken, an insect and the like, and is intended to encompass any subject that participates in a clinical study test, with no disease clinical findings found therein or which participates in epidemiological studies or used as a control group.

Unless otherwise specified, the term "measuring a miRNA or protein level of a gene", as used herein, refers to detecting a target of interest in a corresponding sample.

In the present invention, the target of interest is a corresponding miRNA or protein in a sample. In other words, the detection of the miRNA or protein allows the onset of the cancer to be determined.

For example, a level of the miRNA or protein may be measured in a biological sample from a patient suspicious of cancer.

In the present invention, TUT4 and TUT7 are used as diagnostic markers for determining the onset of cancer on the basis of the finding that a subject with cancer exhibits a significant increase in the expression level of TUT4 and TUT7 in vivo, compared to a subject without cancer.

Meant by the term "gene" in the present invention is any nucleic acid sequence or a portion thereof which plays a functional role in encoding a protein or in transcribing a gene or regulating the expression of a different gene. The gene may consist of an entire nucleic acid responsible for encoding a functional protein or only a portion thereof responsible for encoding or expressing a protein. The nucleic acid sequence may contain a genetic abnormality within exons, introns, initiation or termination regions, promoter sequences, other regulatory sequences or unique adjacent regions to the gene.

In the present invention, the agent for measuring an expression level of mRNA refers to a molecule which is useful for detecting the maker mRNA by determining an expression level of the mRNA whose expression level is increased in biological specimens from a subject affected with cancer.

In detail, the agent for measuring an expression level of a miRNA may be a primer or probe binding specifically to the miRNA.

Descriptions of overlapping contents with the "composition for diagnosis cancer, comprising an agent for measuring miRNA expression levels of miR-324-5p and miR-324-3p.1" will be omitted to avoid excessive complexities.

As used herein, the term "protein" is intended to encompass a fragment, an analog, or a derivative of a given protein, which possesses essentially the same biological activity or function as the given protein.

In the present invention, the agent for measuring a protein level refers to a molecule which is useful for detecting the maker protein by determining an expression level of the protein whose expression level is increased in biological specimens from a subject affected with cancer.

In detail, the agent for measuring a protein level may be an antibody binding specifically to the protein.

In the present invention, the term "antibody" is used in the broadest sense and is intended to encompass an intact monoclonal antibody, an intact polyclonal antibody, a multi-specific (e.g., bispecific) antibody prepared from at least two intact antibodies, and an antibody fragment exhibiting a desired biological activity.

Still another aspect of the present invention pertains to a kit for diagnosing cancer comprising the composition for diagnosing cancer.

The kit may comprise a generally used tool, reagent, and the like suitable for use in a kit for diagnosing cancer in addition to the agent for measuring an mRNA or protein level of the gene.

Descriptions of overlapping contents with the "composition for diagnosing cancer, comprising an agent for measuring miRNA expression levels of miR-324-5p and miR-324-3p.1" will be omitted to avoid excessive complexities.

### Advantageous Effects

The present invention relates to a pharmaceutical composition comprising a TUT4/7 expression regulator for prevention or treatment of cancer. The pharmaceutical composition of the present invention can repress the function of TUT4/7 to prevent cell division and inhibit cancer development and can increase the level of miR-324-5p and downregulate miR-324-3p.1, thus finding applications in effective preventing, treating, or diagnosing cancer.

### Brief Description of the Drawings

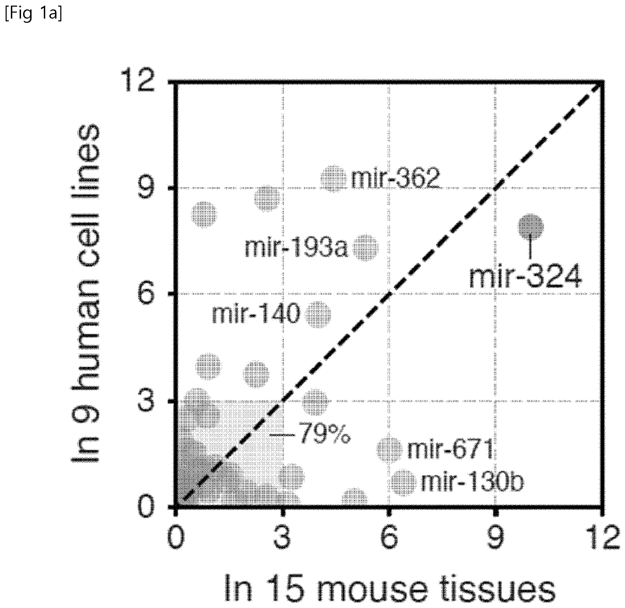

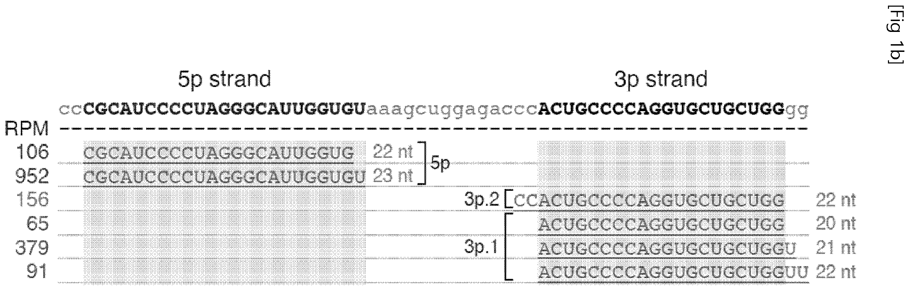

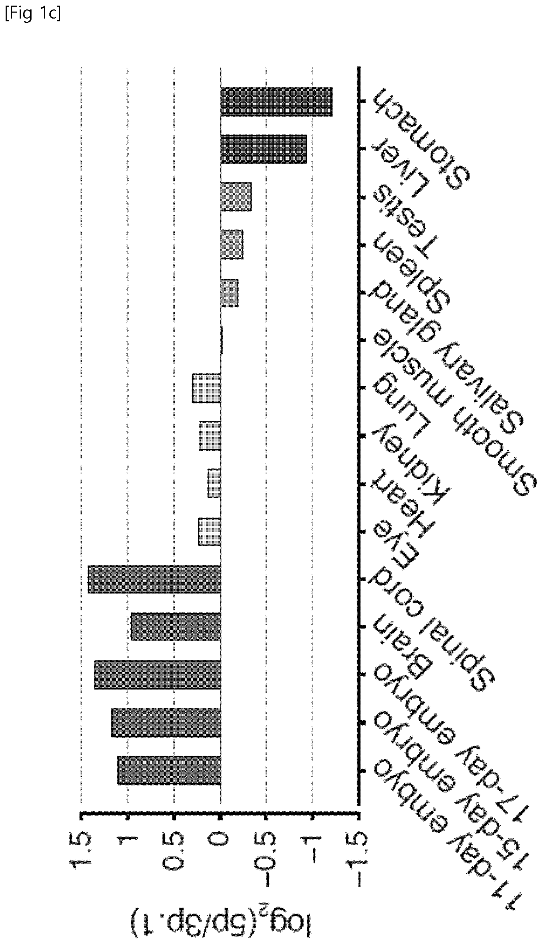

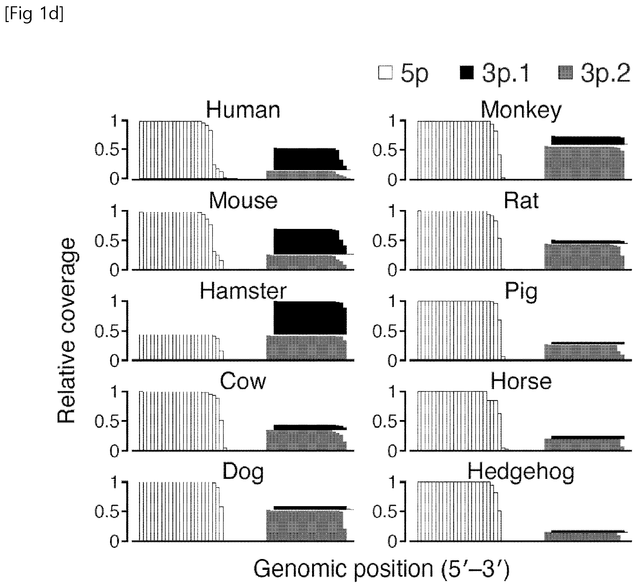

FIG. 1 shows results of arm switching of miR-324: in FIG. 1a: scatter plot of median absolute deviation of the 5p proportion for a miRNA (abundant and ubiquitous miRNAs (>100 median RPM in both datasets, >0 RPM in all samples) were included in this analysis); FIG. 1B: IsomiR profiles of miR-324 in HEK293T cells, uncovered by AQ-seq. RPM-normalized read counts are denoted on the left. Reference sequences of 5p and 3p are marked by gray shade; FIG. 1c: Strand ratios (Sp/3p.1) of miR-324 for the indicated panel of mouse tissues measured by AQ-seq; and FIG. 1d: Conservation of the 5'-isomiRs of miR-324 in mammals.

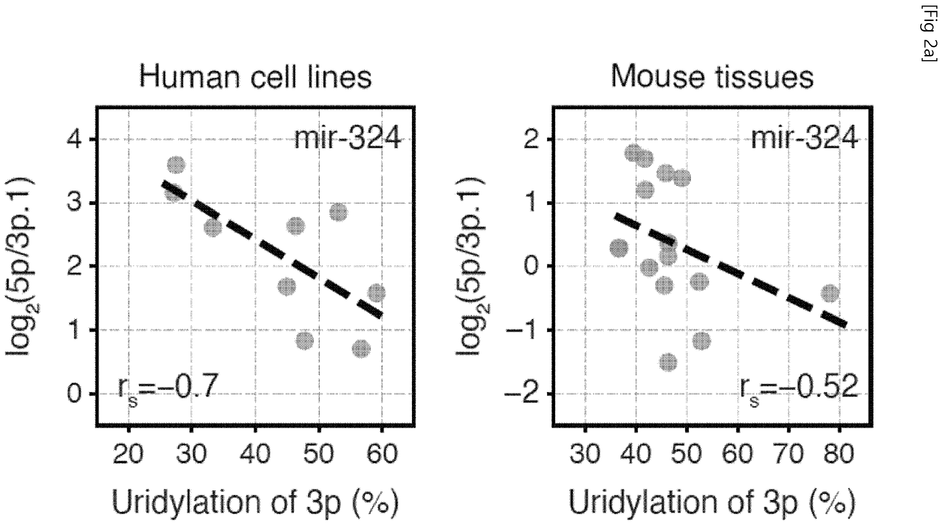

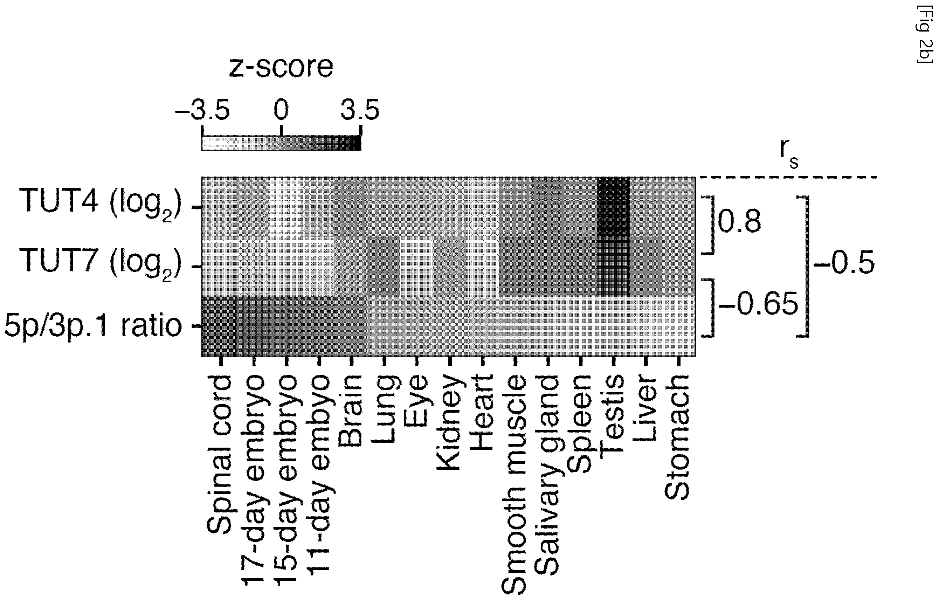

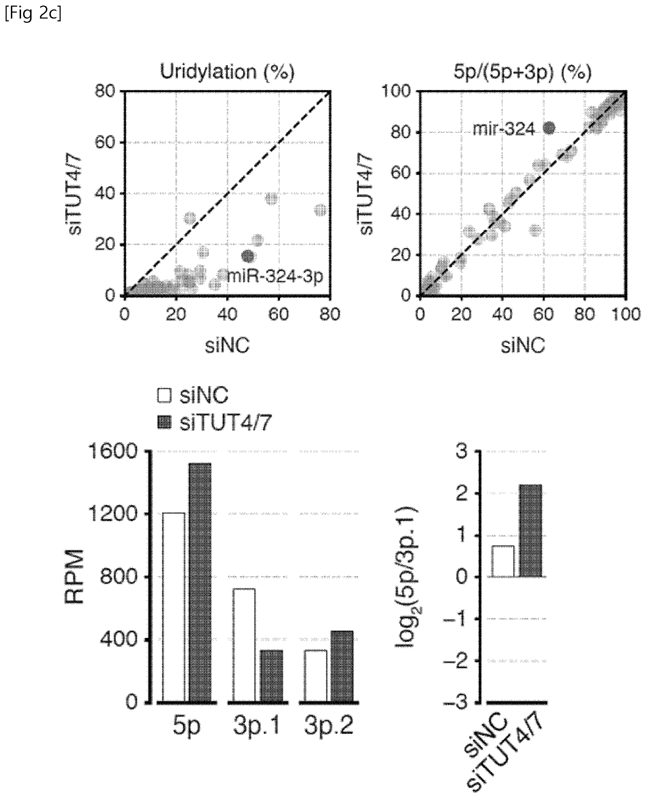

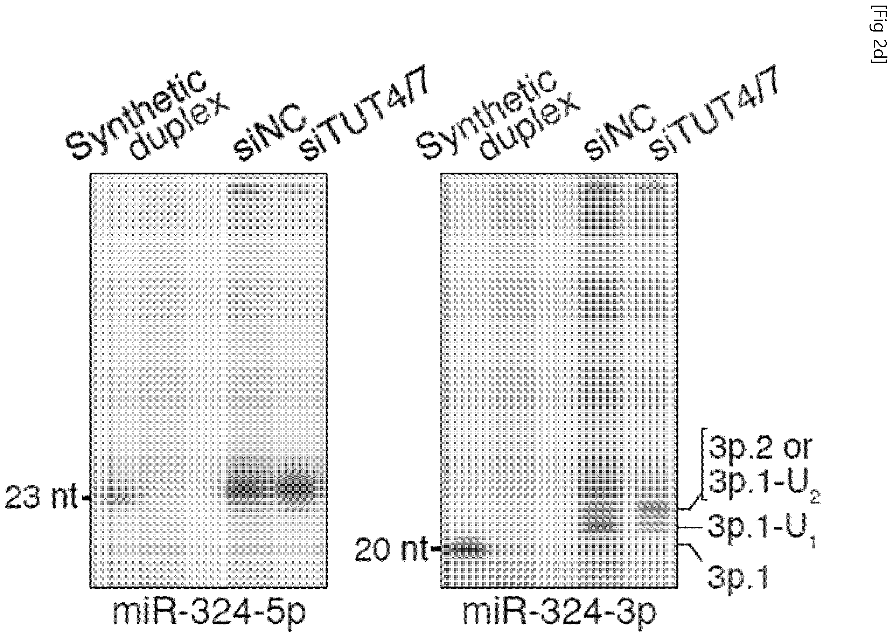

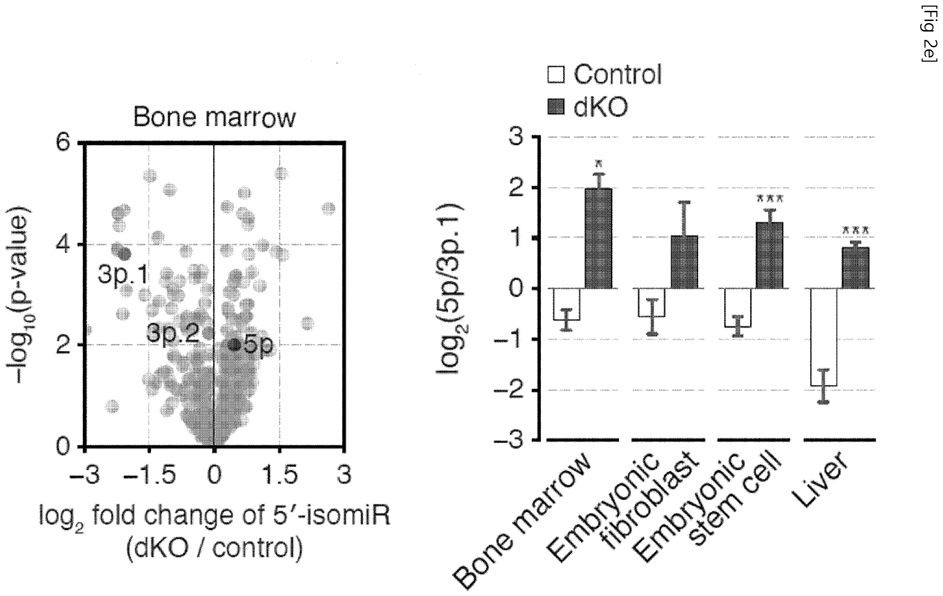

FIG. 2 shows results of miR-324 arm switching controlled by TUT4/7: FIG. 2a: Negative association between the uridylation frequency of miR-324-3p and the 5p/3p.1 ratio of miR-324 in 9 human cell lines and 15 mouse tissues (The linear regression is shown with dashed lines. rₛ, Spearman correlation coefficient); FIG. 2b: The relative TUT4/7 levels and the 5p/3p.1 ratios of miR-324 in the indicated panel of mouse tissues. The TUT4/7 mRNA levels and 5p/3p.1 ratios were quantified by RT-qPCR and AQ-seq, respectively. rₛ, Spearman correlation coefficient; FIG. 2c: AQ-seq results following knockdown of TUT4/7 in HEK293T cells (left and middle: scatter plots of uridylation frequency and the 5p proportion of miRNAs. Abundant miRNAs (>100 RPM) in siNC-transfected sample were included in the analysis. Right: relative abundance of three 5'-isomiRs and log₂-transformed 5p/3p.1 ratio of miR-324)); FIG. 2d: Northern blot of miR-324-5p and miR-324-3p in HEK293T cells (synthetic miR-324 duplex was used for size references); and FIG. 2e: sRNA-seq results following double knockout of Tut4/7 in mice (Left: abundance changes of 5'-isomiRs by TUT4/7 depletion in bone marrow. 5'-isomiRs with RPM over 10 in the control samples were included in this analysis. P-value by the two-sided Student's t test. Right: log₂-transformed 5p/3p.1 ratio of miR-324. Bars indicate mean ± s.d.. Bone marrow, n = 2; embryonic fibroblasts, n = 2; embryonic stem cells, n = 3; control and Tut4/7 dKO in liver, n = 4 and 3, respectively. *p < 0.05, ***p < 0.001 by two-sided Student's t test).

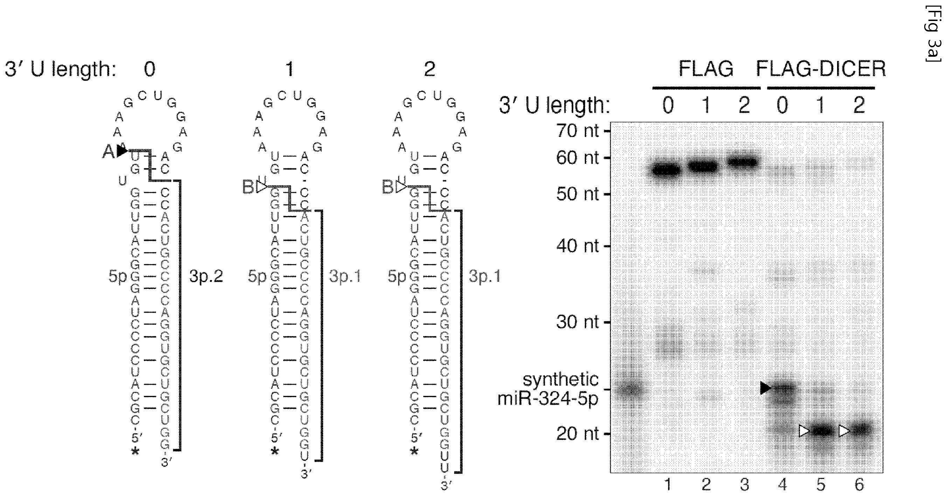

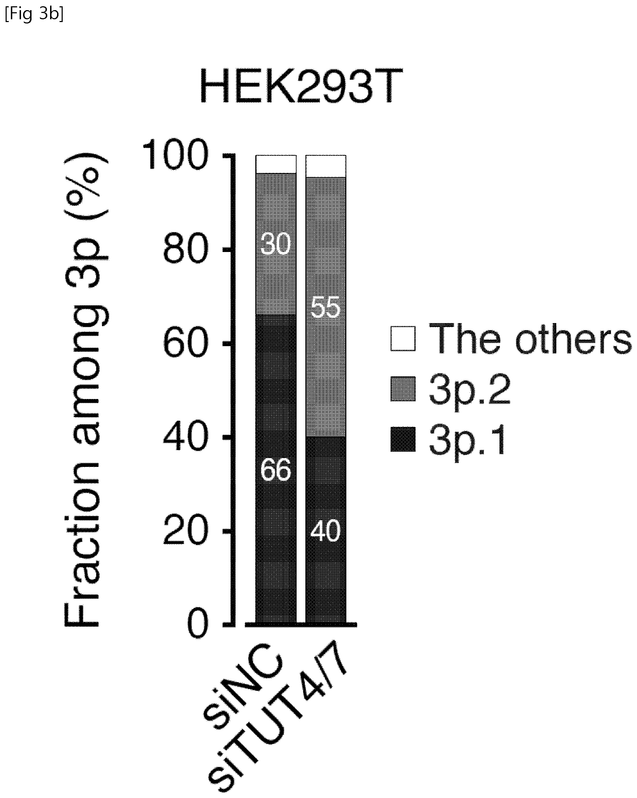

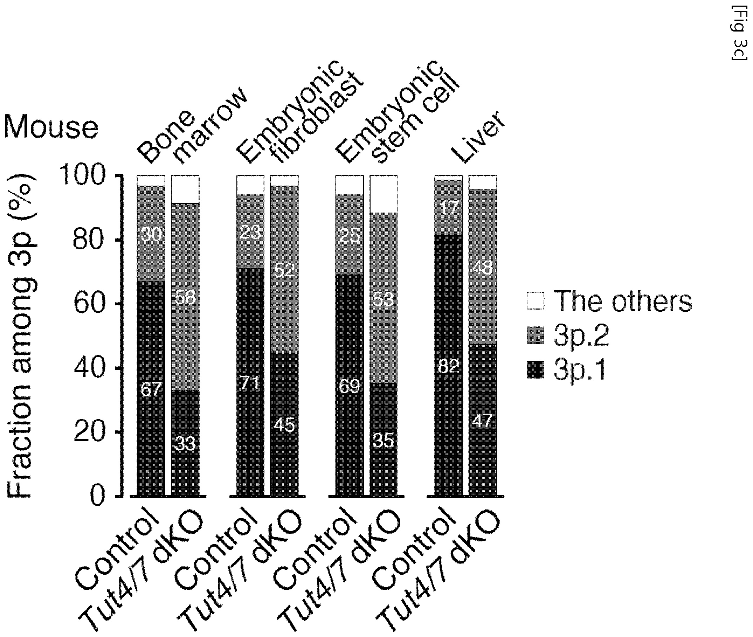

FIG. 3 shows results of uridylation-induced alternative Dicer processing of pre-miR-324: FIG. 3a: *In vitro* processing of unmodified, mono- or di-uridylated forms of pre-miR-324 by immunopurified Dicer (Synthetic miR-324-5p (23 nt), marked in blue, was used as a size control. Major cleavage products and their corresponding cleavage sites are marked with arrowheads. *: radiolabeled 5' phosphates); and FIG. 3b: 5'-isomiR composition of miR-324-3p in HEK293T cells; and FIG. 3c: 5'-isomiR composition of miR-324-3p in mice (Bars indicate mean. Bone marrow, n = 2; embryonic fibroblasts, n = 2; embryonic stem cells, n = 3; control and Tut4/7 dKO in liver, n = 4 and 3, respectively).

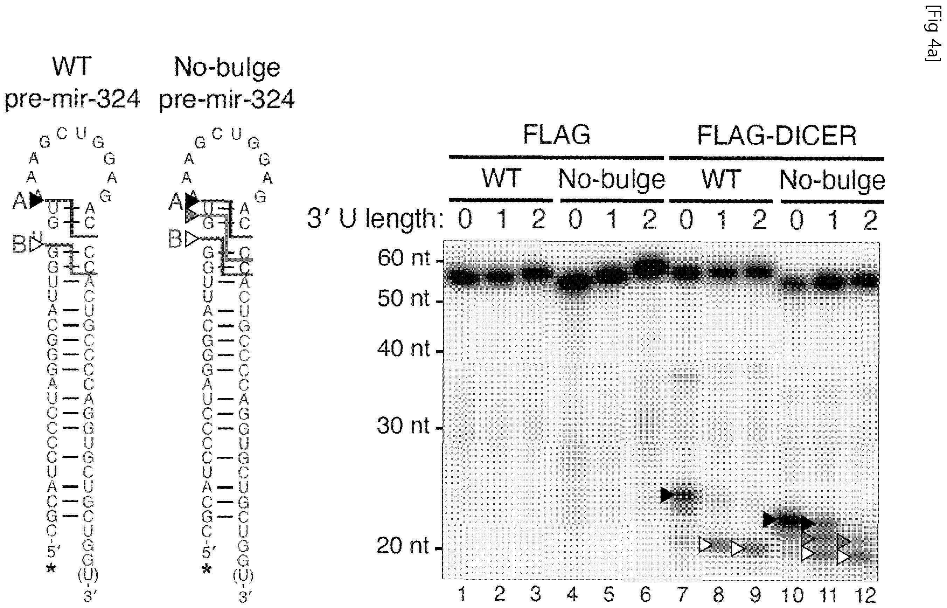

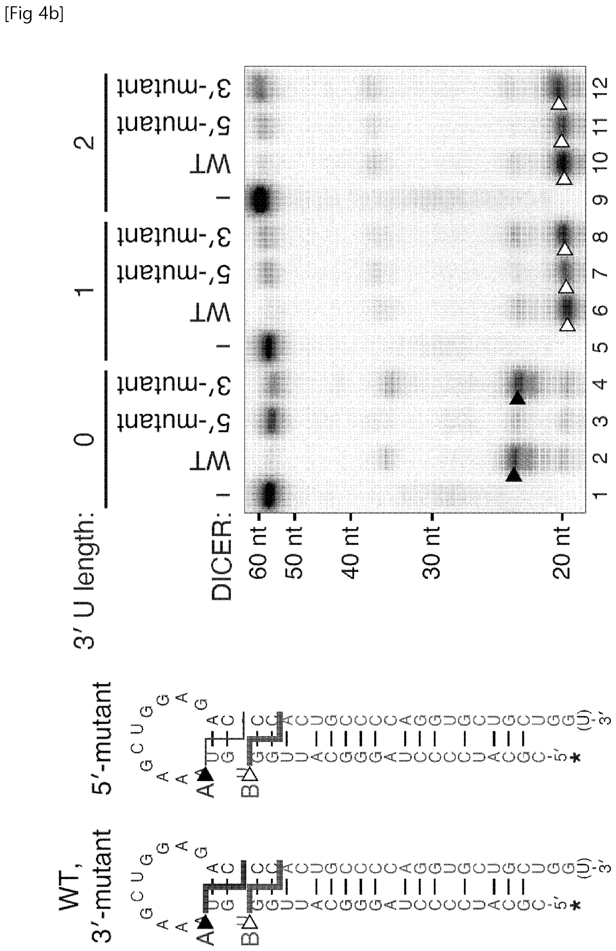

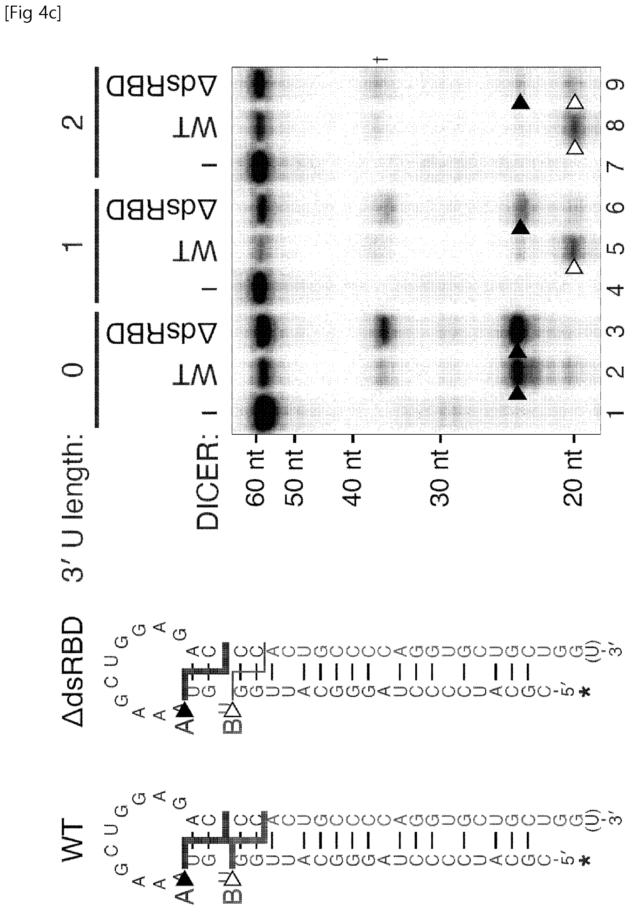

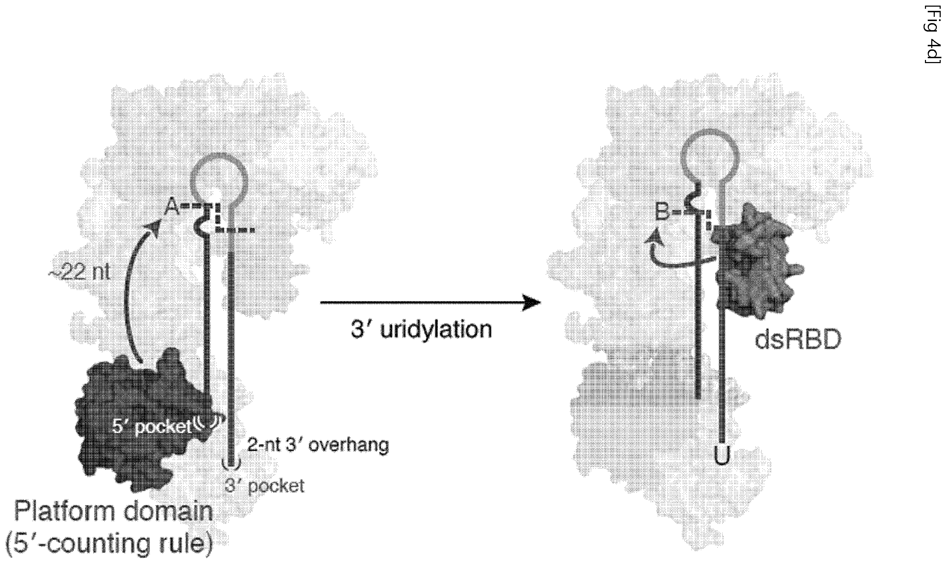

FIG. 4 shows results of alternative Dicer processing facilitated by double stranded RNA-binding domain (dsRBD): FIG. 4a: *In vitro* processing of unmodified, mono- or di-uridylated forms of wild-type or no-bulge mutant pre-miR-324 by immunopurified Dicer; FIGS. 4b and 4c: *In vitro* processing of unmodified, mono- or di-uridylated forms of pre-miR-324 by immunopurified Dicer pocket mutants (4b) or dsRBD-deleted mutant (4c) (Major cleavage products and their corresponding cleavage sites are marked with arrowheads. *: radiolabeled 5' phosphates; †: nicked products at the 3p positions); and FIG. 4d: A proposed model for uridylation-mediated alternative Dicer processing of pre-miR-324.

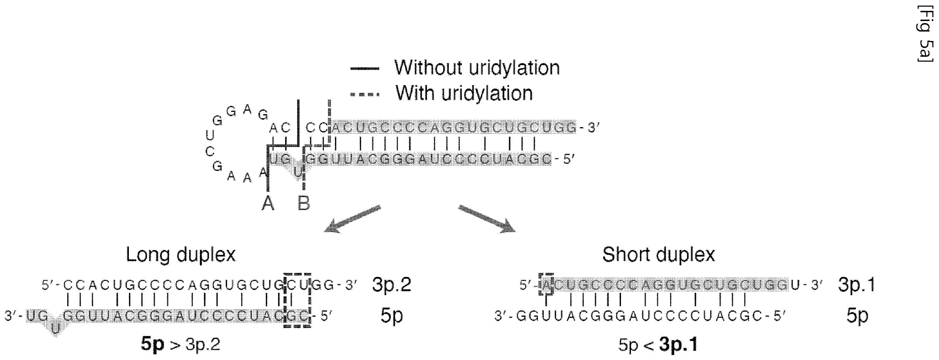

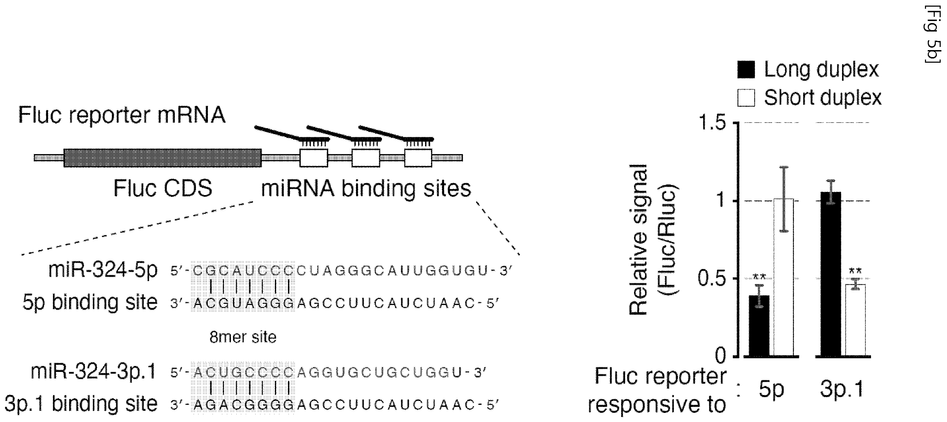

FIG. 5 shows results of alternative Dicer processing-induced arm switching: FIG. 5a: A schematic diagram of strand selection of miR-324 duplexes produced from unmodified and uridylated pre-miR-324 (Dashed rectangles indicate end properties that dictate the strand selection of each duplex); and FIG. 5b: Luciferase reporter assay with two miR-324 duplexes (Left: an illustration of firefly reporter mRNAs that have three 8mer target sites of either miR-324-5p or 3p.1. Right: the relative reporter activity from the firefly luciferase in response to two miR-324 duplexes in HEK293T cells. The reporter activity from Renilla luciferase was used as a control. Bars indicate mean ± s.d. (n = 2, biological replicates) . **p < 0.01 by two-sided Student's t test).

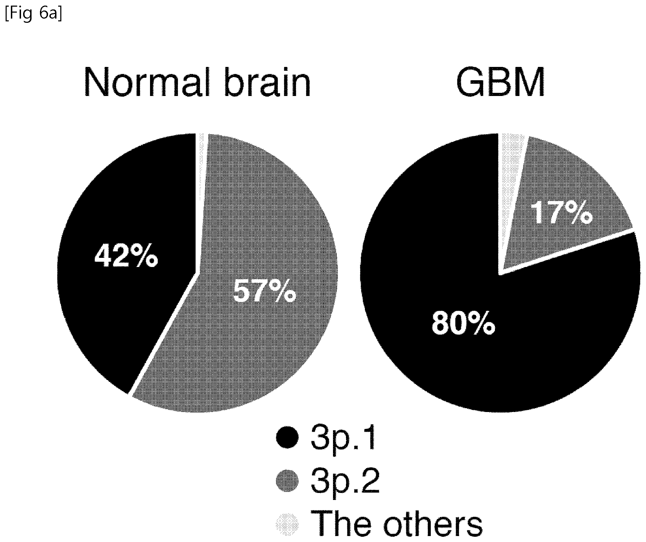

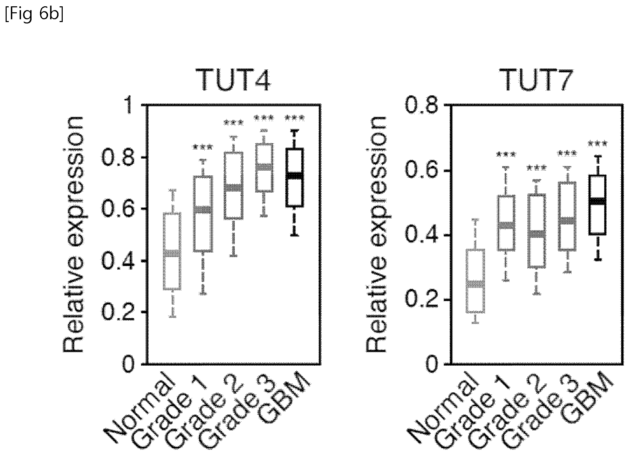

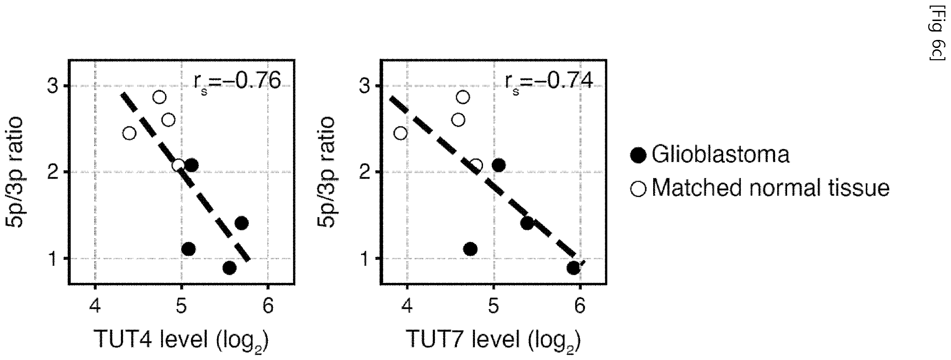

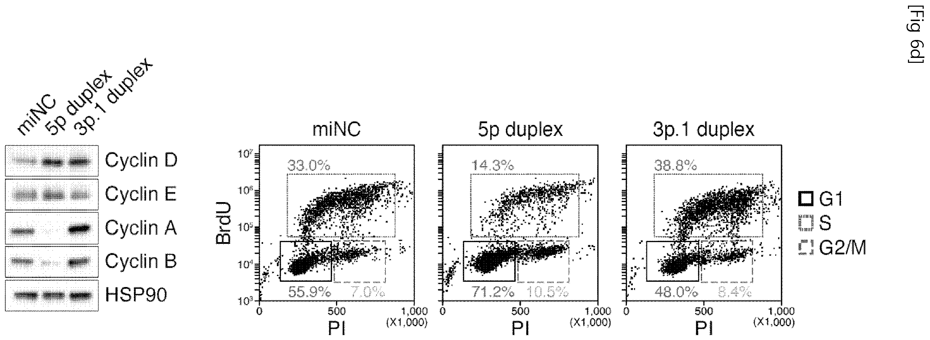

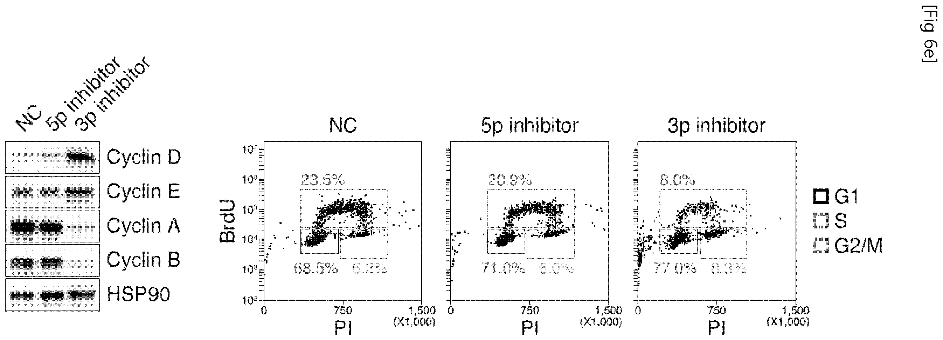

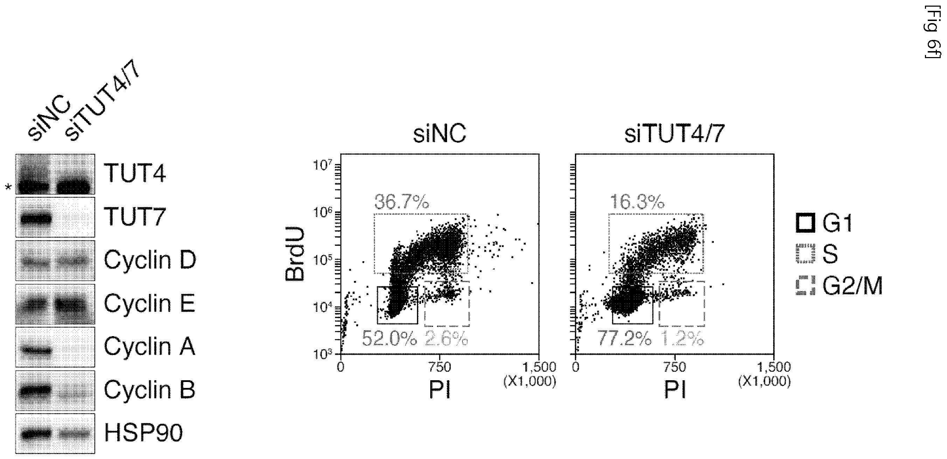

FIG. 6 shows results of the cell cycle progression controlled by miR-324 arm switching: FIG. 6a: ratios of 5'-isomiR compositions of miR-324-3p in glioblastoma and normal brain tissues (normal brain, n = 3; and glioblastoma, n = 6); FIG. 6b: Expression levels of TUT4/7 in normal, lower-grade glioma, and glioblastoma tissues from the Oncopression database (normal, n = 723; grade 1, n = 74; grade 2, n = 133; grade 3, n = 132; glioblastoma, n = 865. ***p < 0.001 by one-way ANOVA with Tukey's post hoc test for multiple comparisons); FIG. 6c: Negative correlation between the TUT4/7 expression level and the miR-324-5p/3p ratio in glioblastoma (black) and matched normal brain tissues (white) (the linear regression is shown with dashed lines. rₛ: Spearman correlation coefficient); FIG. 6d: Western blot of the indicated cyclin proteins and cell cycle profile after overexpressing the synthetic 5p duplex (long duplex) or 3p.1 duplex (short duplex) of miR-324 in A172 cells; FIG. 6e: Western blot of the indicated cyclin proteins and cell cycle profile after inhibiting miR-324 by locked nucleic acid antisense oligonucleotides in A172 cells; and FIG. 6f: Western blot of the indicated proteins and cell cycle profile after knocking down TUT4/7 in A172 cells (*: a cross-reacting band. PI: propidium iodide, BrdU: bromodeoxyuridine).

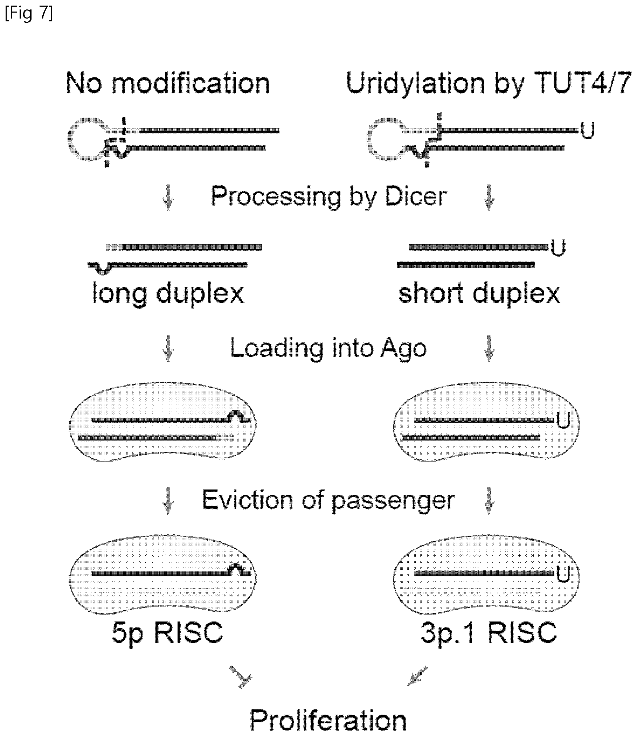

FIG. 7 is a schematic view showing a miR-324 arm switching mechanism model.

### Mode for Carrying Out the Invention

A better understanding of the present invention may be obtained via the following examples which are set forth to illustrate, but are not to be construed as limiting the present disclosure.

Throughout the description, "%" used to indicate a concentration of a specific material refers to (weight/weight) % for solid/solid, (weight/volume) % for solid/liquid, and (volume/volume) % for liquid/liquid unless otherwise stated.

### [PREPARATION EXAMPLES]

### 1. AQ-seq Library

AQ-seq (bias-minimized sRNA-seq) libraries were constructed using total RNAs from fifteen mouse tissues (Mouse Total RNA Master Panel; Takara) as described in the previous study (Kim et al., 2019).

Briefly, 5 mg of total RNAs was mixed with 10 fmole of 30 equi-molar spike-in RNAs (miRNA-like non-human/mouse/frog/fish RNAs used for bias evaluation). Small RNAs were enriched by size fractionation by 15% urea-polyacrylamide gel electrophoresis and sequentially ligated to the randomized adaptor at the 3' and 5' ends. The ligated RNAs were reverse-transcribed using SuperScript III reverse transcriptase (Invitrogen), amplified using Phusion High-Fidelity DNA Polymerase (Thermo Scientific), and subjected to high-throughput sequencing on the MiSeq platform (Illumina)

### 2. High-throughput sequencing of miRNA

Data processing was performed as described in the previous study (Kim et al., 2019) except that reads of the sRNA-seq results from mouse were mapped to the mouse genome (mm10).

Briefly, the 3' adaptor was clipped from the reads using Cutadapt (Martin, 2011). For AQ-seq data, 4-nt degenerate sequences were further removed with FASTX-Toolkit (http://hannonlab.cshl.edu/fastxtoolkit/). After filtering out short, low-quality, and artifact reads using FASTX-Toolkit, AQ-seq data were aligned to the spike-in sequences, followed by mapping the unaligned reads to the genome next, while the other sRNA-seq data were mapped to the genome using BWA (Li and Durbin, 2009) . For a given read, selection was made of an alignment result with the best alignment score allowing a mismatch only at the 3' end. miRNA annotations were retrieved using miRBase release 21 by the intersect tool in BEDTools (Kozomara and Griffiths-Jones, 2014; Quinlan and Hall, 2010).

For quantitative analysis of miRNA strand ratios, the most abundant 5'-isomiR was identified for a given mature miRNA in the most abundantly expressed cell line or tissue. Then, the ratio between the top 5'-isomiRs from 5p and 3p was calculated for all of the cell lines or tissues. Non-repetitive miRNA genes of which both strands were annotated in miRBase release 21 were included in this analysis.

### 3. Targetome Analysis

Results from two modified versions of AGO CLIP-seq (CLEAR-CLIP and CLASH) were analyzed to identify miR-324 targets (Helwak et al., 2013; Moore et al., 2015).

To begin with, for analysis of CLEAR-CLIP, the 3' and 5' adapters were clipped using cutadapt followed by extraction of reads containing miR-324 sequences. Then, the target RNA sequences were mapped to the mouse genome (mm10). Annotations were retrieved using GENCODE release M19 by the intersect tool in BEDTools.

For analysis of CLASH, the supplementary file of the CLASH data generated by the protocol E4 was used. Target genes of miR-324-3p were subdivided according to the 5' end of the miR-324-3p sequences.

### 4. Plasmid construction

To construct plasmids for expression of wild-type, 5' pocket mutant Dicer, and 3' pocket mutant Dicer, the coding sequence of the Dicer reference (RefSeq NM_030621) was amplified with or without mutations introduced in the previous study (Park et al., 2011). The amplified DNAs were subcloned into the pCK-FLAG vector (CMV promoter-driven vector) at the BamHI and XhoI sites using the In-Fusion HD Cloning Kit (Clontech).

For double stranded RNA-binding domain (dsRBD)-deleted Dicer, the coding region except the sequence corresponding to V1849-S1922 amino acids was amplified and subcloned into the pCK-FLAG vector in the same way.

For TRBP, the coding sequence of the human TRBP reference (RefSeq NM_134323) was amplified and subcloned into the pcDNA3-Myc vector (Invitrogen) at the BamHI and XhoI sites.

To construct plasmids for luciferase assay, synthetic DNA oligos containing the three 8mer target sites of either miR-1-3p, miR-324-5p, or miR-324-3p.1 were amplified and inserted into pmiRGLO (Promega) at the XhoI and XbaI sites. The sequences of synthetic DNA oligos and PCR primers are listed in Table 1, below.

**TABLE 1**

| Name | Sequence (5' -> 3') |
|---|---|
| 3X miR-1-3p | AGTTGTTTCAATCTACTTCCGAACATTCCACGCGCAATC TACTTCCGAACATTCCACGCGCAATCTACTTCCGAACAT TCCAAAACGAGC (SEQ ID NO: 23) |
| 3X miR-324-5p | AGTTGTTTCAATCTACTTCCGAGGATGCACGCGCAATC TACTTCCGAGGATGCACGCGCAATCTACTTCCGAGGA TGCAAAACGAGC(SEQ ID NO: 24) |
| 3X miR-324-3p.1 | AGTTGTTTCAATCTACTTCCGAGGGGCAGACGCGCAATC TACTTCCGAGGGGCAGACGCGCAATCTACTTCCGAGGGG CAGAAAACGAGC(SEQ ID NO: 25) |
| miRNA_forward primer | CTCGCTAGCCTCGAGAGTTGTTTCAATCTACTTCC(SEQ ID NO: 26) |
| miR-1-3p_reverse primer | CAGGTCGACTCTAGAGCTCGTTTTGGAATGTTCGG(SEQ ID NO: 27) |
| miR-324-5p_reverse primer | CAGGTCGACTCTAGAGCTCGTTTTGCATCCCTCGG(SEQ ID NO: 28) |
| miR-324-3p.1_reverse primer | CAGGTCGACTCTAGAGCTCGTTTTCTGCCCCTCGG(SEQ ID NO: 29) |

### 5. Cell culture and transfection

A172 and U87MG were obtained from Korean Cell Line Bank. HEK293T, A172, and U87MG were maintained in DMEM (WELGENE) supplemented with 10% fetal bovine serum (WELGENE). Primary tumor cells derived from a glioblastoma patient (TS13-64) were established from fresh glioblastoma tissue specimens, as approved by the institutional review board of Yonsei University College of Medicine (4-2012-0212, 4-2014-0649).

For tumorsphere culture, TS13- 64 cells were grown in DMEM (WELGENE) supplemented with 1X B-27 (Thermo Scientific), 20 ng/mL of bFGF (R&D Systems), and 20 ng/mL of EGF (Sigma-Aldrich) (Kong et al., 2013).

To knock down TUT4/7, cells were transfected twice with 20~22 nM siRNAs using the Lipofectamine 3000 reagent (Thermo Scientific) and harvested 4 days after the first transfection.

For overexpression of Dicer, HEK293T cells were transfected with pCK-FLAG-Dicer using Lipofectamine 3000 and harvested 2 days post transfection.

To deliver synthetic miRNAs or inhibitors, cells were transfected with 20 nM of synthetic miRNA duplexes or 80 nM of LNA miRNA inhibitors using Lipofectamine 3000 and harvested 2 days post transfection.

Control siRNA (AccuTarget negative control siRNA), siRNAs, control miRNA, and synthetic miR-324 duplexes were obtained from Bioneer. Control miRNA inhibitor (miRCURY LNA miRNA inhibitor negative control A) and miR-324 inhibitors (hsa-miR-324-5p and hsa-miR-324-3p miRCURY LNA miRNA inhibitor) were obtained from QIAGEN. The sequences of synthetic siRNAs and miRNAs are listed in Table 2, below.

**TABLE 2**

| Name | Sense strand sequence in each complex(5' -> 3') |
|---|---|
| | (underlined: 3' overhang by deoxyribonucleotides) |
| siTUT4 (duplex binding sequence) | GGUUGCUUCAGACUUUAUATT(SEQ ID NO: 5) (UAUAAAGUCUGAAGCAACCTT)(SEQ ID NO: 9) |
| | GGAAUGAAGAAGAGAAAGATT(SEQ ID NO: 6) (UCUUUCUCUUCUUCAUUCCTT)(SEQ ID NO: 10) |
| | GGAGAAACGACAUAAGAAATT (SEQ ID NO: 7) (UUUCUUAUGUCGUUUCUCCTT) (SEQ ID NO: 11) |
| | GGUUAGAGCUGCUUAAAUUTT (SEQ ID NO: 8) (AAUUUAAGCAGCUCUAACCTT) (SEQ ID NO: 12) |
| siTUT7 (duplex binding sequence) | GAAAAGAGGCACAAGAAAATT(SEQ ID NO: 13) (UUUUCUUGUGCCUCUUUUCTT)(SEQ ID NO: 17) |
| | GCAAAGAGGACAAAGAAAUTT(SEQ ID NO: 14) (AUUUCUUUGUCCUCUUUGCTT)(SEQ ID NO: 18) |
| | GAUAAGUAUUCGUGUCAAATT(SEQ ID NO: 15) (UUUGACACGAAUACUUAUCTT)(SEQ ID NO: 19) |
| | GAACAUGAGUACCUAUUUATT(SEQ ID NO: 16) (UAAAUAGGUACUCAUGUUCTT)(SEQ ID NO: 20) |

| Name | Sequence(5' -> 3') |
|---|---|
| miNC | Sense: 5Phos/CCUACGCCACCAAUUUCGUUU(SEQ ID NO: 30)Antisense: 5Phos/ACGAAAUUGGUGGCGUAGGUU(SEQ ID NO: 31) |
| miR-324 long duplex (5p duplex) | 5p: 5Phos/CGCAUCCCCUAGGGCAUUGGUGU(SEQ ID NO: 1)3p.2: 5phos/CCACUGCCCCAGGUGCUGCUGG(SEQ ID NO: 2) |
| miR-324 short duplex (3p.1 duplex) | 5p: 5Phos/CGCAUCCCCUAGGGCAUUGG(SEQ ID NO: 32)3p.1+U: 5phos/ACUGCCCCAGGUGCUGCUGGU(SEQ ID NO: 33) |
| miR-324-5p | CGCAUCCCCUAGGGCAUUGGUGU(SEQ ID NO: 1) |

### 6. Northern blot

Total RNAs were isolated using TRIzol (Invitrogen) and small RNAs (< 200 nt) were enriched with the miRVana miRNA Isolation Kit (Ambion). Then, the RNAs were resolved on 15% urea-polyacrylamide gels. Synthetic miR-324 duplex and Decade Markers System (Ambion) were used as size markers. RNAs were transferred to Hybond-NX membranes (Amersham) and crosslinked to the membranes with 1-ethyl-3-(3-dimethylaminopropyl)carbodiimide. Antisense probes were radiolabeled at their 5' ends with [γ⁻³²P] ATP by T4 polynucleotide (Takara) and purified using Performa Spin Columns (Edge BioSystems). The membranes were incubated with denatured UltraPure Salmon Sperm DNA Solution (Thermo Scientific) in PerfectHyb Plus Hybridization Buffer, hybridized with antisense probes, and washed with mild wash buffer (0.05% SDS and 2X SSC), followed by stringent wash buffer (0.1% SDS and 0.1X SSC). Radioactive signals were detected by Typhoon FLA 7000 (GE Healthcare) and analyzed using the Multi Gauge software (FujiFilm).

miR-324-3p was detected first, and then miR-324-5p was detected after stripping off the probes. To remove the probes from the blot, the membrane was soaked in preboiled 0.5% SDS for 15 min. Synthetic miR-324 duplex (AccuTarget) was obtained from Bioneer. The sequences of probes are listed in Table 3, below.

**TABLE 3**

| Name | Sequence (5' -> 3') |
|---|---|
| miR-324-5p probe | ACCAATGCCCTAGGGGATGCG(SEQ ID NO: 34) |
| miR-324-3p probe | CAGCAGCACCTGGGGCAGT(SEQ ID NO: 35) |

### 7. Quantitative real-time PCR (RT-qPCR)

To measure mRNA levels in mouse tissues, RNAs from Mouse Total RNA Master Panel (Takara) were reverse-transcribed using RevertAid Reverse Transcriptase (Thermo Scientific) and subjected to quantitative real-time PCR with the Power SYBR Green PCR Master Mix (Thermo Scientific) on StepOnePlus Real-Time PCR System (Thermo Scientific). GAPDH was used as an internal control. The sequences of qPCR primers are listed in Table 4, below.

**TABLE 4**

| Name | Sequence (5' -> 3') |
|---|---|
| TUT4 forward primer | GCCTAAAAGGCGGATAGCCATTG(SEQ ID NO: 36) |
| TUT4 reverse primer | GCTGCTCTGAATCTTTCCACAAC(SEQ ID NO: 37) |
| TUT7 forward primer | CAGCTCAGCAAAAGAGGAGGCA(SEQ ID NO: 38) |
| TUT7 reverse primer | CATAGAACCGCAGCAATTCCACC(SEQ ID NO: 39) |
| GAPDH forward primer | ACCACAGTCCATGCCATCAC(SEQ ID NO: 40) |
| GAPDH reverse primer | TCCACCACCCTGTTGCTGTA(SEQ ID NO: 41) |

To quantify the miR-324-5p/3p strand ratio, total RNAs were isolated using TRIzol (Invitrogen). Then, cDNAs were synthesized using the TaqMan miRNA Reverse Transcription Kit (Applied Biosystems) and subjected to quantitative real-time PCR with the TaqMan MicroRNA Assay (Applied Biosystems) on StepOnePlus Real-Time PCR System (Thermo Scientific). U6 snRNA was used as an internal control.

### 8. In vitro Dicer processing assay

Pre-miR-324 and its variants were radiolabeled with [γ⁻³²P] ATP by T4 polynucleotide kinase and purified using Oligo Clean & Concentrator (Zymo Research) according to manufacturer's instructions.

For immunoprecipitation of FLAG-Dicer, the HEK293T cells overexpressing Dicer proteins were lysed with lysis buffer (500 mM NaCl, 20 mM Tris (pH 8.0), 1 mM EDTA, 1% Triton X-100) and sonicated using Bioruptor Standard (Diagenode). After centrifugation, the supernatant was incubated with 10 µL of ANTI-FLAG M2 Affinity Gel (Sigma-Aldrich). The beads were washed twice with lysis buffer, four times with high salt buffer (800 mM NaCl and 50 mM Tris (pH 8.0)), and four times with buffer D (200 mM KCl, 20 mM Tris (pH 8.0), 0.2 mM EDTA) and then resuspended in 10 µL buffer D.

The immune-purified Dicer was subjected to in vitro reactions in a total volume of 30 µL (containing 2mM MgCl₂, 1mM DTT, SUPERase In RNase Inhibitor (Thermo Scientific) 60 units, 5'-radiolabeled pre-miR-324). The RNAs were purified through phenol extraction or using Oligo Clean & Concentrator (Zymo Research) and resolved on 15% urea-polyacrylamide gels. Synthetic miR-324-5p and Decade Markers System (Ambion) were used as size markers. Synthetic pre-miR-324 fragments were obtained from IDT. Synthetic miR-324-5p was obtained from Bioneer. The sequences of synthetic pre-miR-324 fragments and miR-324-5p are listed in Table 5, below.

**TABLE 5**

| Name | Sequence (5' -> 3') |
|---|---|
| Pre-miR-324 5' half | CGCAUCCCCUAGGGCAUUGGUGUAAAGCUG(SEQ ID NO: 42) |
| No-bulge pre-miR-324 5' half | CGCAUCCCCUAGGGCAUUGGGUAAAGCUG(SEQ ID NO: 43) |
| Pre-miR-324 3' half | 5Phos/GAGACCCACUGCCCCAGGUGCUGCUGG(SEQ ID NO: 44) |
| Pre-miR-324 3' half + U | 5Phos/GAGACCCACUGCCCCAGGUGCUGCUGGU(SEQ ID NO: 45) |
| Pre-miR-324 3' half + UU | 5Phos/GAGACCCACUGCCCCAGGUGCUGCUGGUU(SEQ ID NO: 46) |

### 9. Dual luciferase reporter assay

pmiRGLO containing three 8-mer target sites of either miR-1-3p, miR-324-5p, or miR-324- 3p.1 was co-transfected, together with control miRNA or miR-324 duplexes, into HEK293T cells with the aid of Lipofectamine 3000 (Thermo Scientific). After 2 days of transfection, the cells were harvested and subjected to reporter assay. The reporter activities were measured using Dual-Luciferase Reporter Assay System according to the manufacturer's instructions (Promega) on the Spark microplate reader (TECAN). Given that miR-1-3p is little expressed in HEK293T (~ 8 RPM in the AQ-seq result) (Kim et al., 2019), pmiRGLO with three 8mer target sites of miR-1-3p was used for a plasmid control. Control miRNA was used for further normalization.

### 10. Gene expression analysis of glioblastoma patient data

From Oncopression (http://oncopression.com), Preprocessed gene expression data using microarray were retrieved (Lee and Choi, 2017). The REMBRANDT gene expression dataset (E-MTAB-3073) was obtained from ArrayExpress (Madhavan et al., 2009).

For analysis of simultaneous profiling of mRNAs and miRNAs (Gulluoglu et al., 2018), pre-processing microarray data were normalized by the robust multi-array average (RMA) using the limma package in R before being used for gene expression analysis.

### 11. Survival analysis

Level 3 miRNA gene quantification data and clinical data for TCGA glioblastoma patients were obtained from the GDC legacy archive and the GDC data portal, respectively. The patients were stratified according to the miR-324-5p/3p ratio and the top and bottom 40% of the cases were included in the analysis. The patient's survival was estimated by the Kaplan-Meier method and tested by the two-sided log-rank test using the survival package in R.

### 12. Western blot

Cells were washed with PBS and lysed in RIPA buffer (Thermo Scientific) supplemented with Protease Inhibitor Cocktail Set III (Merck Millipore) and Phosphatase Inhibitor Cocktail II (AG Scientific). Protein concentrations were measured using the BCA Protein Assay Kit (Pierce Biotechnology) and equal amounts of proteins were separated on 4-12% Tris-Glycine Gels (Thermo Scientific) and transferred onto Immobilon-P PVDF membranes (Merck Millipore). Membranes were incubated with 5% skimmed milk in PBS-T (PBS (Amresco) + 0.1% Tween 20 (Anatrace) ) and then treated with primary antibodies. After being washed three times with PBS-T, the membranes were incubated with HRP-conjugated secondary antibodies. The protein bands were detected by SuperSignal West Pico PLUS Chemiluminescent Substrate (Thermo Scientific) and scanned by ChemiDoc XRS+ System (Bio-Rad) .

Rabbit polyclonal antibodies against TUT4 (18980-1-AP, RRID:AB_10598327, 1:500) and TUT7 (25196-1-AP, 1:500) were obtained from Proteintech. A mouse monoclonal antibody against Cyclin E (sc-247, RRID:AB_627357, 1:1,000) and rabbit polyclonal antibodies against Cyclin A (sc-751, RRID:AB_631329, 1:1,000), Cyclin B1 (sc-752, RRID:AB_2072134, 1:1,000), and Cyclin D1 (sc-753, RRID:AB_2070433, 1:1,000) were purchased from Santa Cruz Biotechnology. A rabbit polyclonal antibody against HSP90 (4874, RRID:AB_2121214, 1:1,000) was purchased from Cell Signaling. HRP-conjugated goat polyclonal antibodies against rabbit IgG (111-035-144, RRID:AB_2307391, 1:10,000) and mouse IgG (115-035-146, RRID:AB_2307392, 1:10,000) were purchased from Jackson ImmunoResearch.

### 13. Flow cytometry

After being incubated with 10 µM BrdU for 3-8 hours, cells were fixed by ice-cold 70% ethanol. The fixed cells were incubated with FITC-conjugated anti-BrdU antibody (11-5071-42, RRID: AB_11042627, Invitrogen) and further stained with 20 µg/mL of propidium iodide (Sigma-Aldrich) in the presence of 10 pg/mL of RNase A (Thermo Scientific) before detection on BD Accuri C6 Plus Flow Cytometer. Cell cycles were analyzed by the BD Accuri C6 system software.

### [EXAMPLES]

### 1. Alternative strand selection (or Arm switching) of miR-324

First, search was made for miRNAs that exhibit significant alterations in their strain ratio. For accurate quantification of the strand ratio, the AQ-seq library protocol was employed. The variation of strand selection was estimated across 15 different mouse tissues/developmental stages (FIG. 1a, X axis). In addition, a sRNA-seq dataset from 9 human cell lines was used to compare strand use in humans.

As shown in FIG. 1a, the majority of miRNAs in human and mouse datasets are invariable in strand ratio (ca. 79%, median variance below 3%). Nevertheless, several cases distinctively displaying variations, such as miR-324, miR-362, miR-193a, and miR-140, were identified.

Particularly, a focus was made on miR-324 because the arm switching of miR-324 was considered to have a substantial effect on cellular fate decision.

As shown in FIGS. 1b to 1d, three groups of 5'-isomiRs (5p, 3p.1, and 3p.2) that are produced from the single miR324 locus were detected (FIG. 1b). The 5p strand is dominant in mouse embryos and neuronal tissues while the major 3p isoform 3p.1 is prevalent in the liver and stomach (FIG. 1c). Furthermore, the 5'-isomiR groups are found in mammals, suggesting that the mechanism for alternative maturation is evolutionarily conserved (FIG. 1d).

### 2. miR-324 arm switching is controlled by TUT4 and TUT7.

The uridylation frequency of 3p correlates negatively with the 5p/3p.1 ratio (FIG. 2a), leading to the hypothesis that uridylation may be involved in the arm switching.

Meanwhile, the terminal uridylyl transferases TUT4 (also known as ZCCHC11 and TENT3A) and TUT7 (also known as ZCCHC6 and TENT3B) are reported to catalyze uridylation of diverse RNA species, including a specific set of pre-miRNAs (e.g., let-7 precursors). TUT4 and TUT7 (TUT4/7) act redundantly on most substrates.

On the basis of the facts in the foregoing, quantitation (RT-qPCR) of TUT4/7 levels in mouse tissues resulted in the finding that the 5p/3p.1 ratio is higher in cell types where TUT4 and TUT7 levels are relatively low (FIG. 2b).

To examine whether TUT4/7 is involved in miR-324 maturation, TUT4/7 was depleted in HEK293T sRNA-seq (AQ-seq) was performed.

As shown in FIG. 2c, TUT4/7 knockdown reduced uridylation of miRNAs, including miR-324-3p (left upper panel). In addition, TUT4/7 knockdown resulted in a change in isoform composition of miR-324, such as a reduction in 3p.1 and an increase in 5p and the minor isoforms (5p/3p.1) increased, with the consequent increase of a ratio between the major isoforms (5p/3p.1) (right upper and lower panels).

These sequencing data were consistent with the northern blot (FIG. 2d), indicating that there is a change in the quantity of miR-324 isoforms upon TUT4/7 knockdown.

In addition, as can be seen in FIG. 2e, re-analysis of the published sRNA-seq data from the TUT4/7 double knockout mice shows that 3p.1 level decreases whereas the 5p level increases upon the knockout (left panel). Consequently, a change in the strand ratio by TUT4/7 double knockout was observed in all tissues/cell types examined (bone marrow, embryonic fibroblasts, embryonic stem cells, and liver) (right panel).

### 3. Uridylation induces alternative Dicer processing of pre-miR-324.

TUT4/7 are known to modify pre-miRNAs rather than mature miRNAs. In order to understand how TUT4/7 modulate miR-324 strand selection, the effect of uridylation on pre-miRNA processing was examined by performing *in vitro* Dicer processing assays with synthetic pre-miR-324.

As shown in FIG. 3a, when pre-miR-324 carries one or two extra uridine residues at the 3' end, the Dicer processing site is shifted by 3-nt (from position A to position B in FIG. 3a). Unmodified pre-miR-324 mainly releases a longer duplex containing 5p and 3p.2 (black arrowheads) whereas uridylated pre-miR-324 is cleaved at the alternative position (position B) to yield a shorter duplex consisting of shorter 5p and 3p.1 (white arrowheads). Thus, TUT4/7-mediated uridylation of pre-miR-324 leads to alternative Dicer processing.

Moreover, as shown in FIGS. 3b and 3c, the sequencing data from TUT4/7-depleted human and mouse cells showed that the relative abundance of the 3p isomiRs (3p.1 vs. 3p.2) changed on TUT4/7 knockdown (FIG. 3b) and knockout (FIG. 3c), confirming the conclusion that uridylation alters Dicer cleavage site selection.

### 4. Alternative Dicer processing is facilitated by the double-stranded RNA binding domain.

First, in order to identify the determinant responsible for the 3-nt shift, U bulge was removed from pre-miR-324.

For the "no-bulge" mutant, as shown in FIG. 4a, Dicer no longer induced the abrupt shift to position B upon uridylation, but rather caused single-nucleotide gradual shifts (lanes 10-12). Hence, the U bulge is an anti-determinant for cleavage at sites between positions A and B and serves as the ci-acting element responsible for alternative processing.

Next, examination was made to reveal relationship between the 5'- and 3'-counting rules and the pre-miR-324 processing. In this regard, Dicer mutants in the 5' or 3' pocket were utilized.

As can be seen in FIG. 4b, the 5' pocket mutant Dicer failed to cleave pre-miR-324 at position A while the 3' pocket mutant exhibited slight affection on processing efficiency without an effect on cleavage site choice. This result indicates the importance of the 5' pocket for cleavage at position A.

Moreover, an investigation was made of the contribution of other domains in Dicer. For use in testing the role of the Dicer dsRBD *in vitro,* a dsRBD deletion mutant was generated.

As shown in FIG. 4c, the dsRBD deletion brought about a significant change in the processing pattern. That is, without the dsRBD, Dicer cleaved mainly at position A, which implies that the dsRBD is responsible for the cleavage at position B.

Taken together, unmodified pre-miR-324 is cleaved at position A, relying on the 5' pocket existing in the platform domain of Dicer as shown in FIG. 4D (left panel). However, when uridylated, the end structure (3-to 4-nt 3' overhang) is not tightly fixed to the platform/PAZ domains so that pre-miR-324 is repositioned in Dicer with the aid of the dsRBD, resulting in alternative processing at position B (right panel).

### 5. Alternative Dicer processing leads to arm switching.

Taken together, the data obtained from the Examples showed that, as can be seen in FIG. 5a, pre-miR-324 can yield two alternative forms of duplexes, that is, a long duplex (position A; 5p/3p.2) and a short duplex (position B; 5p/3p.1).

In order to examine strand selection in cells, reporters containing the 3' UTR complementary to miR-324-5p or miR-324-3p.1 was constructed (left panel in FIG. 5b), and co-transfected, along with synthetic duplexes ((5p/3p.2) or (5p/3p.1)). As a result, the long duplex selectively repressed the reporter with the 5p complementary sites but not with 3p.1 sites. As opposed to the long duplex, the short duplexes downregulated the 3p.1 reporter but not the 5p reporter (the right panel in FIG. 5b).

These results imply that 5p and 3p.1 are indeed differentially produced from the long duplex and the short duplex, respectively. That is, from the long duplex, the 5p strand is selected because the 5' end of 5p is thermodynamically unstable compared with that of 3p.2 (blue dashed rectangle in FIG. 5a). In contrast, in the short duplex, 3p.1 starts with a 5' adenosine, which is favored by AGO (red dashed rectangle in FIG. 5a).

Taken together, the results demonstrate that the arm switching of miR-324 results from the alternative Dicer processing.

### 6. miR-324 arm switching affects cell-cycle progression.

The 5' end of miR-324-3p was previously reported to vary in human glioblastoma (GBM). The proportion of 3p.1 is substantially greater in tumor than in normal brain tissue, as shown in FIG. 6a.

SO, to examine the possibility that TUT4/7-mediated miR-324 maturation is differentially modulated in the GBM context, transcriptome datasets were analyzed using Oncopression.

As can be seen in FIG. 6b, TUT4 and TUT7 are significantly upregulated in GBM.

In addition, using an independent dataset that profiled both mRNAs and miRNAs, comparison with normal samples was carried out to corroborate the upregulation of TUT4/7 levels in GBM and a reduction of the miR-324-5p/3p ratio in GBM.

As shown in FIG. 6c, TUT4/7 levels negatively correlate with 5p/3p ratios. These data suggest that TUT4/7-mediated miR-324 regulation may be physiologically relevant in GBM.

On the basis of the facts in the foregoing, to understand the function of miR-324 arm switching, the molecular and cellular phenotypes driven by perturbation of miR-324 strand selection in GBM cell lines was investigated.

As shown in FIGS. 6D and 6E, Transfection of the synthetic long duplex ("5p duplex") or an antisense oligo against 3p ("3p inhibitor") resulted in an accumulation of cyclins D and E and a reduction in cyclins A and B (left panels). In addition, cell-cycle profiling indicated that the cells are arrested at the G1 phase (right panels).

As shown in FIG. 6f, TUT4/7 knockdown also gave rise to cyclin dysregulation and G1 arrest.

Taken together, the data indicate that miR-324 isomiRs have opposing functions in GBM cell proliferation.

### [Discussion]

As shown in FIG. 7, the terminal uridylyl transferases TUT4/7 function as key players in the alternative strand selection of miR-324 by uridylating pre-miR-324. That is, TUT4/7 uridylates pre-miR-324, which leads to shifting Dicer cleavage positions on pre-miR-324 to change ratios among the three functionally different microRNAs (one 5p and two 3p isoforms). Therefore, inhibition of uridylation-mediated processing of miR-324 can repress the expression of cell division-associated genes relevant to the growth of glioblastoma.

Being capable of regulating the alternative strand selection of microRNA(miR-324) by TUT4/7, the present invention can be effectively applied to the prevention and treatment of cancer (encephaloma).

### Industrial Applicability

The present invention relates to a pharmaceutical composition comprising a TUT4/7 expression regulator for prevention or treatment of cancer and, more specifically, to a pharmaceutical composition comprising a nucleic acid sequence regulatory of TUT4/7 expression for prevention or treatment of cancer.

## Claims

1. A pharmaceutical composition for preventing or treating cancer, the composition comprising at least one selected from the group consisting of:
a microRNA (miRNA) comprising the nucleotide sequences of SEQ ID NOS: 1 and 2;
a nucleic acid composed of the nucleotide sequence of SEQ ID NO: 4; and
a small interfering RNA (siRNA) comprising at least one nucleotide sequence selected from the group consisting of SEQ ID NOS: 5 to 20.

2. The pharmaceutical composition of claim 1, wherein the microRNA is in a duplex form in which a microRNA comprising the nucleotide sequence of SEQ ID NO:1 and a microRNA comprising the nucleotide sequence of SEQ ID NO: 2 are bound to each other.

3. The pharmaceutical composition of claim 1, wherein the small interfering RNA is in a complex form in which: a small interfering RNA comprising any one of the nucleotide sequences of SEQ ID NOS: 5 to 8 and a small interfering RNA comprising any one of the nucleotide sequences of SEQ ID NOS: 9 to 12 bind to each other; or a small interfering RNA comprising any one of the nucleotide sequences of SEQ ID NOS: 13 to 16 and a small interfering RNA comprising any one of the nucleotide sequences of SEQ ID NOS: 17 to 20 bind to each other

4. The pharmaceutical composition of claim 1, wherein the cancer is selected from the group consisting of encephaloma, colon cancer, large intestine cancer, lung cancer, liver cancer, stomach cancer, esophageal cancer, pancreatic cancer, gallbladder cancer, kidney cancer, bladder cancer, prostate cancer, testis cancer, uterine cervical cancer, endometrial cancer, choriocarcinoma, ovarian cancer, breast cancer, thyroid cancer, brain cancer, head and neck cancer, and malignant melanoma.

5. The pharmaceutical composition of claim 1, wherein the cancer is encephaloma.

6. A composition for diagnosing cancer, comprising an agent for measuring an expression level of at least one miRNA selected from the group consisting of miR-324-5p and miR-324-3p.1.

7. The composition of claim 6, wherein the agent comprises a primer or a probe which bind specifically to the at least one miRNA.

8. A kit for diagnosing cancer, comprising the composition of any one of claims 6 and 7.

9. The kit of claim 8, wherein the kit is for use in RT-PCR, real-time RT-PCR, northern blotting, RNA protection assay, or a microarray chip.

10. A composition for diagnosing cancer, comprising an agent for measuring an mRNA or protein level of at least one gene selected from the group consisting of TUT4 (terminal uridylyl transferases 4) and TUT7 (terminal uridylyl transferases 7).

11. The composition of claim 10, wherein the agent for measuring an mRNA level comprises a primer or a probe which bind specifically to at least one mRNA of TUT4 and TUT7.

12. The composition of claim 10, wherein the agent for measuring a protein level comprises an antibody binding specifically to at least one protein of TUT4 and TUT7.

13. A kit for diagnosing cancer, comprising the composition of any one of claims 10 to 12.

14. The kit of claim 13, wherein the kit is for use in RT-PCR, a DNA chip, or a protein chip.


<110>     Seoul National University R&DB Foundation
          INSTITUTE FOR BASIC SCIENCE

<120>     Pharmaceutical Composition Comprising Modulator of TUT4/7
          Expression

<130>     PP200013

<150>     KR 10-2019-0022551
<151>     2019-02-26

<160>     46

<170>     KoPatentIn 3.0

<210>     1
<211>     23
<212>     RNA
<213>     Artificial Sequence

<220>
<223>     miR-324-5p


<400>     1
cgcauccccu agggcauugg ugu                                               23


<210>     2
<211>     22
<212>     RNA
<213>     Artificial Sequence

<220>
<223>     miR-324-3p.2


<400>     2
ccacugcccc aggugcugcu gg                                                22


<210>     3
<211>     20
<212>     RNA
<213>     Artificial Sequence

<220>
<223>     miR-324-3p.1


<400>     3
acugccccag gugcugcugg                                                   20


<210>     4
<211>     18
<212>     DNA
<213>     Artificial Sequence

<220>
<223>     miR-324-3p inhibitor

<400>    4
cagcagcacc tggggcag                                                    18


<210>    5
<211>    21
<212>    DNA_RNA
<213>    Artificial Sequence

<220>
<223>    siTUT4 duplex


<220>
<223>    3-overhang with deoxyribonucleotides are TT


<400>    5
gguugcuuca gacuuuauat t                                                21


<210>    6
<211>    21
<212>    DNA_RNA
<213>    Artificial Sequence

<220>
<223>    siTUT4 duplex


<220>
<223>    3-overhang with deoxyribonucleotides are TT


<400>    6
ggaaugaaga agagaaagat t                                                21


<210>    7
<211>    21
<212>    DNA_RNA
<213>    Artificial Sequence

<220>
<223>    siTUT4 duplex


<220>
<223>    3-overhang with deoxyribonucleotides are TT


<400>    7
ggagaaacga cauaagaaat t                                                21


<210>    8
<211>    21
<212>    DNA_RNA
<213>    Artificial Sequence

<220>
<223>    siTUT4 duplex

<220>
<223>    3-overhang with deoxyribonucleotides are TT


<400>    8
gguuagagcu gcuuaaauut t                                                  21


<210>    9
<211>    21
<212>    DNA_RNA
<213>    Artificial Sequence

<220>
<223>    siTUT4 duplex


<220>
<223>    3-overhang with deoxyribonucleotides are TT


<400>    9
uauaaagucu gaagcaacct t                                                  21


<210>    10
<211>    21
<212>    DNA_RNA
<213>    Artificial Sequence

<220>
<223>    siTUT4 duplex


<220>
<223>    3-overhang with deoxyribonucleotides are TT


<400>    10
ucuuucucuu cuucauucct t                                                  21


<210>    11
<211>    21
<212>    DNA_RNA
<213>    Artificial Sequence

<220>
<223>    siTUT4 duplex


<220>
<223>    3-overhang with deoxyribonucleotides are TT


<400>    11
uuucuuaugu cguuucucct t                                                  21


<210>    12
<211>    21
<212>    DNA_RNA

<213>    Artificial Sequence

<220>
<223>    siTUT4 duplex


<220>
<223>    3-overhang with deoxyribonucleotides are TT


<400>    12
aauuuaagca gcucuaacct t                                                21


<210>    13
<211>    21
<212>    DNA_RNA
<213>    Artificial Sequence

<220>
<223>    siTUT7 duplex


<220>
<223>    3-overhang with deoxyribonucleotides are TT


<400>    13
gaaaagaggc acaagaaaat t                                                21


<210>    14
<211>    21
<212>    DNA_RNA
<213>    Artificial Sequence

<220>
<223>    siTUT7 duplex


<220>
<223>    3-overhang with deoxyribonucleotides are TT


<400>    14
gcaaagagga caaagaaaut t                                                21


<210>    15
<211>    21
<212>    DNA_RNA
<213>    Artificial Sequence

<220>
<223>    siTUT7 duplex


<220>
<223>    3-overhang with deoxyribonucleotides are TT


<400>    15
gauaaguauu cgugucaaat t                                                21

<210>    16
<211>    21
<212>    DNA_RNA
<213>    Artificial Sequence

<220>
<223>    siTUT7 duplex


<220>
<223>    3-overhang with deoxyribonucleotides are TT


<400>    16
gaacaugagu accuauuuat t                                                21


<210>    17
<211>    21
<212>    DNA_RNA
<213>    Artificial Sequence

<220>
<223>    siTUT7 duplex


<220>
<223>    3-overhang with deoxyribonucleotides are TT


<400>    17
uuuucuugug ccucuuuuct t                                                21


<210>    18
<211>    21
<212>    DNA_RNA
<213>    Artificial Sequence

<220>
<223>    siTUT7 duplex


<220>
<223>    3-overhang with deoxyribonucleotides are TT


<400>    18
auuucuuugu ccucuuugct t                                                21


<210>    19
<211>    21
<212>    DNA_RNA
<213>    Artificial Sequence

<220>
<223>    siTUT7 duplex


<220>

<223>    3-overhang with deoxyribonucleotides are TT

<400>    19
uuugacacga auacuuauct t                                                21

<210>    20
<211>    21
<212>    DNA_RNA
<213>    Artificial Sequence

<220>
<223>    siTUT7 duplex

<220>
<223>    3-overhang with deoxyribonucleotides are TT

<400>    20
uuugacacga auacuuauct t                                                21

<210>    21
<211>    4938
<212>    RNA
<213>    Artificial Sequence

<220>
<223>    Terminal uridylyl transferases 4

<400>    21
auggaagagu cuaaaaccuu aaaaagugaa aaucaugaac caaagaagaa uguuauuugu      60

gaagaaagca aagcaguuca aguuauaggu aaucaaacau ugaaagcuag aaaugauaaa     120

uccguaaaag aaauugagaa cagcucucca aauaggaaua guaguaaaaa aaauaagcaa     180

aaugauauuu guauagaaaa aacagaaguu aaaucaugua aaguaaaugc ugccaaccuu     240

ccagguccua aagauuuggg guuaguccuu cgagaucaaa gucauugcaa agcaaaaaaa     300

uuuccuaauu caccggugaa agccgaaaag gcaaccauuu cacaggcaaa aucagaaaag     360

gcaaccaguu uacaggcaaa agcagaaaaa ucaccaaagu caccuaauuc agugaaagca     420

gaaaaagcau ccaguuauca gaugaaguca gaaaaaguac caaguucacc agcagaagca     480

gaaaagggac ccaguuuacu guugaaagac augagacaga agacagaauu acaacagauu     540

ggaaaaaaaa uuccaagcuc cuuuacuucu guggacaaag ugaauauuga agcuguaggg     600

ggagaaaaau gugcucugca aaacucacca cgaucucaga agcaacagac auguacagau     660

aauacuggug auucugauga uagugcuuca ggaauugaag acguaucgga cgauuuaagu     720

aaaaugaaga augaugaauc uaauaaagaa aauucuucag agauggacua cuuagaaaau     780

gccacuguga uagaugaauc ugcauugaca ccugagcaga ggcugggguu gaaacaagca     840

gaagaacgcu uagaaagaga ucacaucuuu cgacuugaaa aacgaucacc agaauauacc     900

aauugucggu aucuaugcaa acuuugcuua auucacauug aaaauaucca gggggcucau 960

aaacauauaa aggagaaacg acauaagaaa aauauuuugg aaaaacaaga agaaagugag 1020

cuucguucuc ugccaccucc uuccccugcc cacuuggcug cuuuaagugu ugcagucauu 1080

gaauuagcaa aagaacaugg aauaacagau gaugaccuca gaguccguca ggaaauugug 1140

gaggaaaugu caaagguuau aacgacauuu uuaccagaau guucacuuag guuguauggc 1200

ucaucucuga cuagauuugc ucugaaaagu agugauguua auauagauau aaaauuuccu 1260

cccaagauga aucauccaga ucuucugaua aaaguacuug ggauuuuaaa gaaaaaugua 1320

uuauauguag auguggaauc ugauuuucac gcuaaaguuc cuguuguggu gugcagagau 1380

cgaaaaagug guuuacuuug uagagugagu gcaggaaacg auauggcaug ucucacuacu 1440

gauuuacuua cugcccuugg caaaauagaa ccugucuuua uucccuuggu guuagccuuu 1500

cgcuacuggg cuaaguugug cuauauugac ucccaaacug augguggaau cccuucuuac 1560

uguuuugcuu uaauggugau guuuuuucua caacagagaa aacccccucu ucuuccuugc 1620

uuacuuggaa guuggauuga aggcuuugac ccaaaaagaa uggaugacuu ucagcugaag 1680

ggcauaguag aagagaaguu ugugaagugg gaauguaauu caaguagugc aacugagaaa 1740

aacucaauug cugaggaaaa caaagcuaag gcagaccaac caaaagauga uaccaagaag 1800

acagaaacag acaaccaaag uaaugccaug aaggaaaaac auggcaaauc uccuuuagca 1860

uuggaaacac caaaucgggu auccuuggga caguuauggu uagagcugcu uaaauucuac 1920

acacuggauu uugcuuugga ggaauauguc auauguguac ggauacaaga uauuuuaaca 1980

agagaaaaua aaaacuggcc uaaaaggcga auagccauug aagauccauu uucagucaag 2040

aggaauguug cacggagcuu aaacagccag cugguuuacg aauauguugu ggagagauuu 2100

agggcagcuu aucgguauuu ugccuguccu cagacgaagg guggaaauaa gucuacagug 2160

gauuucaaga aaagagagaa ggggaaaaua agcaauaaga aaccagucaa gucgaacaau 2220

auggcaacca augguuguau ucugcuuggg gaaaccacag aaaaaauaaa ugcagaaaga 2280

gagcaaccug uucaauguga ugaaauggac uguacaucac agagauguau uauugacaac 2340

aacaauuugu ugguaaauga acuagauuuu gcugaccacg gacaggacuc uucaucucuu 2400

ucuaccagca aaagcaguga aauagagcca aaauuagaua agaaacaaga ugauuuagcg 2460

ccuucagaaa cuuguuuaaa aaaagagcuc agccaaugua auugcauuga uuugucuaag 2520

ucgccugacc cagauaaauc uacuggaaca gacugcaggu caaauuuaga aacagagagu 2580

ucacaucaga gugugugcac cgacacaucu gcuaccucuu gcaacugcaa agcuacagaa 2640

gaugcuucug accuuaauga ugaugauaac cuccccaccc aggaauuaua uuauguguuu 2700

gauaaguuua uuuuaaccuc uggcaagcca ccaacgauag uaugcagcau cugcaaaaag 2760

gauggccauu caaagaauga uugcccagag gauuuuagga aaauugauuu aaaaccucua 2820

ccaccaauga caaaccgauu ucgggaaaua cuugauuuag uauguaaaag auguuuugau 2880

gaguuaucac caccuuguuc ugaacaacac aacagggagc aaauuuuaau uggcuuggaa 2940

aaguuuauuc aaaaagaaua ugaugaaaag gcaagguugu gcuuauuugg cucuucuaag 3000

aauggauuug gauuucguga uagugaucug gauauuugua ugacccugga aggccaugaa 3060

aaugcagaga aauuaaauug uaaggaaaua auugaaaauu uggcaaaaau ucuuaagaga 3120

cauccagguu uaagaaacau uuugccuaua acuacugcca aagugccuau aguaaaauuu 3180

gaacacaggc gaaguggguu agaaggcgau aucaguuuau auaauacguu ggcucaacau 3240

aacacaagaa ugcuagcuac uuaugcagcu auugauccua gagugcagua uuugggauau 3300

acuaugaaag uguuugcuaa gcgaugugac auuggggaug cuuccagggg aaguuuaucu 3360

ucauaugcau auauccuuau ggugcuguac uuucugcagc agagaaagcc accuguuauc 3420

ccaguucuac aagagaucuu ugauggaaaa cagauuccac agagaauggu ugauggaugg 3480

aaugcuuucu ucuuugauaa aacagaagaa cugaaaaagc guuuaccuuc acuuggaaag 3540

aacacagaau cauuagggga gcuuuggcug ggacugcuuc guuucuauac ugaagaguuu 3600

gauuucaagg aauauguaau uagcauucgg cagaaaaagc uguugacaac uuuugagaag 3660

caguggacuu ccaagugcau ugcaauugaa gacccuuuug acuugaauca uaaccuuggu 3720

gcuggaguuu ccagaaaaau gaccaauuuc aucaugaaag cauuuaucaa uggaaggaaa 3780

cuuuuuggua ccccuuuuua uccacucauu ggcagagaag cugaguacuu cuuugauucc 3840

agaguauuaa cagauggaga acuggcuccc aaugauagau guugccgugu guguggaaaa 3900

auaggccacu acaugaaaga cugcccuaaa aggaaaagca guuuacuguu uagacuaaag 3960

aagaaagaca gugaagaaga gaaggaaggg aaugaagaag agaaagauuc ccgagauguu 4020

cuugaccccc gagaccucca cgauacucga gacuuuagag acccgagaga ccucagaugu 4080

uuuauaugug gagaugcugg acauguacga agggagugcc cagaggucaa gcuggcccgu 4140

cagaggaaua gcaguguggc agcagcccag cugguccgca accuuguaaa ugcucaacag 4200

guggcugguu cagcucagca acagggugau caguccauaa ggacuagaca gucaucagaa 4260

uguucugaau caccaucuua uucuccucag ccucagccau uuccacagaa cucuucccag 4320

ucagcugcua uuacucagcc uucaucucag ccaggauccc aaccuaagcu uggcccaccu 4380

cagcagggag cccaaccucc ccaucagguc cagaugccac uguauaacuu uccccaguca 4440

ccaccagcuc aguauucucc caugcacaau augggauugu ugccaaugca cccucuccag 4500

aucccugccc cguccuggcc cauccauggc ccagugaucc acucugcacc aggcagugcc 4560

cccagcaaua uuggccuaaa ugaucccagc aucaucuuug cacagccugc ugccagaccu 4620

guggcaaucc cuaacacguc ucacgaugga cacuggcccc guacuguggc uccaaauucc 4680

cugguaaaca guggugcagu ggggaauuca gagccaggcu uucgaggacu gacuccucca 4740

auuccuuggg aacaugcacc gcguccccau uucccccuug ucccagcuuc guggccuuau 4800

gguuugcauc aaaacuucau gcaucaggga aaugcccgau uccagcccaa caaaccuuuc 4860

uauacucaag acagaugugc cacccgucgg uguagagagc guugucccca cccaccaaga 4920

ggaaacgugu cggaguaa 4938

<210> 22
<211> 4488
<212> RNA
<213> Artificial Sequence

<220>
<223> Terminal uridylyl transferases 7

<400> 22
augggagaua cagcaaaacc uuauuucgug aagcgcacua aagaccgggg gacuauggau 60

gaugaugacu ucagaagggg ucacccccaa caagauuauu uaauaauaga ugaccaugcu 120

aaaggccaug gcaguaaaau ggaaaagggc cuucaaaaaa agaagauaac accagggaac 180

uaugggaaua cccccagaaa aggaccaugu gcuguuucca gcaauccaua ugcauuuaaa 240

aacccaaucu acagucaacc cgcuuggaug aaugacagcc acaaagauca gaguaagaga 300

uggcugucug augaacauac ugguaauuca gacaacugga gagaauucaa accuggaccu 360

agaauuccug uuauaaaccg acaaagaaaa gacuccuuuc aagaaaauga agaugguuau 420

agguggcaag acacaagagg cugcagaacu guaagacgac uguuucauaa agaccuaaca 480

agccuagaaa ccacgucaga aauggaagca ggaaguccug aaaacaagaa gcagaggucc 540

agaccuagga agccacggaa gacuagaaau gaggaaaaug agcaggaugg agacuuggaa 600

ggcccuguga ucgaugaguc uguacuuuca acgaaggagc ugcuaggcuu acagcaggcu 660

gaggagagac ugaagagaga cugcauugac aggcuaaaaa ggcgaccacg aaacuacccu 720

acagcaaagu acaccugcag acucugugau guuuuaauug aauccauugc auuugcccau 780

aagcauauca aggaaaagag gcacaagaaa aacauuaagg agaagcaaga ggaagaguug 840

cucacuacgu uacccccacc aacacccucc cagauaaaug caguuggcau ugccauugac 900

aaagugguac aggaauuugg cuuacacaau gagaacuugg aacagaggcu ggaaauuaaa 960

cguaucaugg aaaauguguu ccaacacaag uuaccagauu guucccuaag auuauauggg 1020

ucauccugua gcagauuggg uuucaaaaau ucggauguaa acauugacau ccaguuucca 1080

gccauuaugu cucagccaga uguccucuua cuuguucaag aauguuuaaa gaacagugac 1140

uccuuuauug auguugaugc agacuuccau gcuagggugc cagugguggu gugcagagaa 1200

aagcaaagug gucuucugug uaaagugagc gcaggaaaug aaaaugcuug ucugacaaca 1260

aagcauuuaa cugcccuugg aaaacuagaa ccaaagcugg uuccuuuggu gauugcauuu 1320

agguacuggg caaagcuuug caguauagau cgcccugaag aaggaggucu gccaccuuau 1380

guguuugccc ugauggccau uuucuuucuu cagcagagga aagaaccccu uuugccugua 1440

uaucuaggau cauggauuga aggauucuca uuaagcaaac uagggaauuu caaccuucaa 1500

gacauugaaa aagauguugu gaucugggaa cauacugaca gugcugcagg ggacacaggc 1560

auaacaaaag aagaggcacc aagagaaacg ccgauuaaaa ggggacaggu gucauuaaua 1620

uuggauguga aacaccagcc uucaguacca guugggcagc ucugguggaa auugcugcgg 1680

uucuaugcuu uagaauuuaa uuuggcugau uuagugauaa guauucgugu caaagaauug 1740

guaucucggg aauugaagga uuggcccaaa aagcgcauug ccauugaaga ucccuacucu 1800

guuaaaagaa auguggcaag aacccuaaau agucaaccug uguuugaaua uauacuucau 1860

uguuuaagga caacauacaa guauuuugcu cuuccacaca aaauuacaaa auccagccuu 1920

cuaaagccuc ugaaugcaau uacauguauu ucagaacauu cuaaagaagu aauaaaucau 1980

cauccagaug uacaaacaaa agaugauaag cucaaaaacu caguuuuggc ccaagguccu 2040

ggugcuacca guucagcugc aaauaccugu aagguacagc cacuuacucu uaaagagacu 2100

gcugaaaguu uuggaagccc accaaaagaa gaaauggaaa augaacacau caguguccac 2160

ccugaaaacu cagacuguau ccaagcagau guuaacucug augauuacaa gggugauaaa 2220

guauaccauc cagaaacagg aaggaaaaac gagaaagaga aaguuggaag gaagggcaag 2280

caucuguuga cuguugauca gaaacgugga gagcauguug ucuguggcag cacacguaau 2340

aaugagucag agagcacuuu ggauuuagaa ggcuuccaaa aucccacagc uaaagagugu 2400

gagggacuug ccacuuuaga uaacaaggcu gaucuugaug gagaaaguac agaagguacu 2460

gaggaacuag aagacucucu aaaccacuuu acccacucag uacagggcca gacaucagaa 2520

augauucccu cugaugaaga ggaggaggac gacgaagaag aggaggagga agaagaaccu 2580

aggcucacca uuaaccaaag ggaagaugaa gauggcaugg cuaaugaaga ugaguuagac 2640

aacaccuaca cugggucagg ggaugaggac gcccuaucug aagaggauga ugaguuaggc 2700

gaagcugcua aguaugaaga cgugaaagaa uguggaaaac auguagaaag agcucuccua 2760

guggaacuua auaaaauaag ucucaaggaa gaaaauguau gugaagaaaa aaauucaccu 2820

guggaucagu cugauuuuuu uuaugaauuc aguaaacuua ucuucaccaa aggcaagucu 2880

ccuacgguag ugugcagcuu augcaaacga gaggucaucu uaaagaagga cuguccugaa 2940

gacuucaaaa gaauccagcu agaaccucug ccaccauuaa cacccaaguu uuuaaauauc 3000

uuagaucaag ucuguaucca guguuauaag gauuuuucuc caacaauuau agaagaucag 3060

gcucgugaac auauucggca aaaccuagaa aguuucauaa gacaggacuu uccaggaacu 3120

aaauugagcc uguuuggcuc cuccaaaaau ggauuugggu ucaaacagag ugaccuugac 3180

gucuguauga caauuaaugg acuugaaacu gcugagggau uggacugugu cagaacuauu 3240

gaagaauuag caagaguccu cagaaaacau ucaggucuga gaaacaucuu accuauuaca 3300

acagcaaagg ugccaauugu gaaguucuuc cauuugagaa guggucugga aguagauauc 3360

aguuuguaua acacauuggc ccuucauaac acaaggcuuu uaucugcuua uuccgccauu 3420

gaucccagag ugaaguauuu gugcuauacc augaaaguau uuacaaagau gugugauauu 3480

ggugaugcau cuagaggcag cuuaucaucg uaugcauaua cucuuauggu gcuauauuuu 3540

cuccagcaga ggaauccacc agucauuccu guccuucaag agauauacaa aggugaaaag 3600

aaaccugaaa uauuuguuga uggcuggaau auuuauuuuu uugaucaaau agaugaacug 3660

ccuaccuauu ggucagaaug uggaaaaaau acagaaucug uugggcaguu augguugggc 3720

cuucuucguu ucuacacaga ggaauuugau uuuaaagaac auguuauuag caucaggaga 3780

aaaagucugc uuacaacuuu uaagaaacag uggaccucaa aauacauugu uauugaagau 3840

cccuuugauu ugaaucauaa ucuuggagcu ggauuaucaa ggaaaaugac aaauuuuaua 3900

augaaggcuu uuaucaaugg uagaagagua uuugguauuc cugucaaggg auuuccaaag 3960

gacuaccccu caaaaaugga auacuuuuuu gauccagaug uguuaacuga aggagagcug 4020

gccccaaaug auagauguug ucgaauuugu ggaaaaaucg gacacuucau gaaggacugu 4080

ccuaugagga gaaaaguaag acggcggcga gaucaggaag augcccugaa ccaaagauac 4140

ccugagaaca aggaaaaaag aagcaaagag gacaaagaaa uucacaacaa guacacagaa 4200

agggaggugu caacaaaaga agauaagccc auacagugca caccucagaa agccaagcca 4260

augcgggcag cugcugaccu ggggagggag aagauccuca ggccaccagu agaaaaaugg 4320

aagagacagg augacaaaga cuuaagagaa aaacguuguu uuauuugugg aagagaaggg 4380

cacauuaaaa aggaaugccc acaguuuaaa ggcucuucag guagccuuuc caguaaauau 4440

augacucagg gaaaagccuc agcgaagagg acccagcagg aaucauga 4488

<210> 23
<211> 90
<212> RNA
<213> Artificial Sequence

<220>
<223> 3X miR-1-3p

<400> 23
agttgtttca atctacttcc gaacattcca cgcgcaatct acttccgaac attccacgcg 60

caatctactt ccgaacattc caaaacgagc 90

<210> 24

<211>    90
<212>    RNA
<213>    Artificial Sequence

<220>
<223>    3X miR-324-5p


<400>    24
agttgtttca atctacttcc gagggatgca cgcgcaatct acttccgagg gatgcacgcg        60

caatctactt ccgagggatg caaaacgagc                                          90


<210>    25
<211>    90
<212>    RNA
<213>    Artificial Sequence

<220>
<223>    3X miR-324-3p.1


<400>    25
agttgtttca atctacttcc gaggggcaga cgcgcaatct acttccgagg ggcagacgcg        60

caatctactt ccgaggggca gaaaacgagc                                          90


<210>    26
<211>    35
<212>    DNA
<213>    Artificial Sequence

<220>
<223>    miRNA_forward primer


<400>    26
ctcgctagcc tcgagagttg tttcaatcta cttcc                                    35


<210>    27
<211>    35
<212>    DNA
<213>    Artificial Sequence

<220>
<223>    miR-1-3p_reverse primer


<400>    27
caggtcgact ctagagctcg ttttggaatg ttcgg                                    35


<210>    28
<211>    35
<212>    DNA
<213>    Artificial Sequence

<220>
<223>    miR-324-5p_reverse primer

<400>    28
caggtcgact ctagagctcg ttttgcatcc ctcgg                                35

<210>    29
<211>    35
<212>    DNA
<213>    Artificial Sequence

<220>
<223>    miR-324-3p.1_reverse primer

<400>    29
caggtcgact ctagagctcg ttttctgccc ctcgg                                35

<210>    30
<211>    21
<212>    RNA
<213>    Artificial Sequence

<220>
<223>    miNC Sense

<400>    30
ccuacgccac caauuucguu u                                               21

<210>    31
<211>    21
<212>    RNA
<213>    Artificial Sequence

<220>
<223>    miNC Antisense

<400>    31
acgaaauugg uggcguaggu u                                               21

<210>    32
<211>    20
<212>    RNA
<213>    Artificial Sequence

<220>
<223>    3p.1 duplex_5p

<400>    32
   cgcauccccu agggcauugg                                                  20

<210>    33
<211>    21
<212>    RNA
<213>    Artificial Sequence

<220>

<223>    3p.1 duplex_3p.1+U

<400>    33
acugccccag gugcugcugg u                                                21

<210>    34
<211>    21
<212>    DNA
<213>    Artificial Sequence

<220>
<223>    miR-324-5p probe

<400>    34
accaatgccc taggggatgc g                                                21

<210>    35
<211>    19
<212>    DNA
<213>    Artificial Sequence

<220>
<223>    miR-324-3p probe

<400>    35
cagcagcacc tggggcagt                                                   19

<210>    36
<211>    23
<212>    DNA
<213>    Artificial Sequence

<220>
<223>    TUT4 forward primer

<400>    36
gcctaaaagg cggatagcca ttg                                              23

<210>    37
<211>    23
<212>    DNA
<213>    Artificial Sequence

<220>
<223>    TUT4 reverse primer

<400>    37
gctgctctga atctttccac aac                                              23

<210>    38
<211>    22
<212>    DNA
<213>    Artificial Sequence

<220>
<223>    TUT7 forward primer


<400>    38
cagctcagca aaagaggagg ca                                                22


<210>    39
<211>    23
<212>    DNA
<213>    Artificial Sequence

<220>
<223>    TUT7 reverse primer


<400>    39
catagaaccg cagcaattcc acc                                               23


<210>    40
<211>    20
<212>    DNA
<213>    Artificial Sequence

<220>
<223>    GAPDH forward primer


<400>    40
accacagtcc atgccatcac                                                   20


<210>    41
<211>    20
<212>    DNA
<213>    Artificial Sequence

<220>
<223>    GAPDH reverse primer


<400>    41
tccaccaccc tgttgctgta                                                   20


<210>    42
<211>    30
<212>    RNA
<213>    Artificial Sequence

<220>
<223>    Pre-mir-324 5′ half


<400>    42
cgcauccccu agggcauugg uguaaagcug                                         30


<210>    43
<211>    29

<212>    RNA
<213>    Artificial Sequence

<220>
<223>    No-bulge pre-mir-324 5′ half


<400>    43
   cgcaucccuu agggcauugg guaaagcug                                          29


<210>    44
<211>    27
<212>    RNA
<213>    Artificial Sequence

<220>
<223>    Pre-mir-324 3′ half


<400>    44
gagacccacu gccccaggug cugcugg                                           27


<210>    45
<211>    28
<212>    RNA
<213>    Artificial Sequence

<220>
<223>    Pre-mir-324 3′ half + U


<400>    45
gagacccacu gccccaggug cugcuggu                                          28


<210>    46
<211>    29
<212>    RNA
<213>    Artificial Sequence

<220>
<223>    Pre-mir-324 3′ half + UU


<400>    46
gagacccacu gccccaggug cugcugguu                                         29